# EUROPÄISCHE PATENTSCHRIFT

(11) **EP 2 538 980 B1**
(45) Veröffentlichungstag und Bekanntmachung des Hinweises auf die Patenterteilung: **08.04.2015**
(21) Anmeldenummer: 11702402.6
(22) Anmeldetag: 04.02.2011
(51) Int. Cl.: C12N 5/00, A61K 47/48

(54) **VERNETZER FÜR HYDROGELE, DIE SPALTBARE PEPTIDE SOWIE KURZKETTIGE POLYMERE ENTHALTEN**
CROSS-LINKING AGENTS FOR HYDROGELS, CONTAINING CLEAVABLE PEPTIDES AND SHORT-LENGTH POLYMERS
AGENTS RÉTICULANTS POUR HYDROGELS, CONTENANT DES PEPTIDES CLIVABLES ET DES POLYMÈRES À CHAÎNE COURTE

(30) Priorität: 23.02.2010 DE 102010009876
(43) Veröffentlichungstag der Anmeldung: 02.01.2013
(73) Patentinhaber: Cellendes GmbH, 72770 Reutlingen (DE)
(72) Erfinder: WURST, Helmut, 72793 Pfullingen (DE); LARBI, Karima, 218815 Singapore (SG); HERRMANN, Markus, 72770 Reutlingen (DE)
(74) Vertreter: Schwahn, Hartmut
(86) Internationale Anmeldenummer: PCT/EP2011/000503
(87) Internationale Veröffentlichungsnummer: WO 2011/103961

(56) Entgegenhaltungen:
- S KIM & K HEALY: "Synthesis and characterization of injectable poly (N-isopropylacrylamide-co-acrylic acid) hydrogels wiith proteolytically degradable cross-links", BIOMACROMOLECULES., Bd. 4, Nr. 5, 2003, Seiten 1214-1223, XP002635236, ACS, WASHINGTON, DC. ISSN: 1525-7797
- DATABASE BIOSIS [Online] BIOSCIENCES INFORMATION SERVICE, PHILADELPHIA, PA, US; Dezember 2007 (2007-12), KHELFALLAH NAWEL S ET AL: "Design, synthesis, and degradation studies of new enzymatically erodible poly(hydroxyethyl methacrylate)/poly(ethylene oxide) hydrogels", XP002635255, Database accession no. PREV200800472961
- N S KHELFALLAH ET AL: "Synthesis of a new PHEMA-PEO enzymatically biodegradable hydrogel", MACROMOLECULAR RAPID COMMUNICATIONS., Bd. 27, Nr. 13, 5. Juli 2006 (2006-07-05), Seiten 1004-1008, XP002635237, Weinheim (DE).

## Beschreibung

Die Erfindung betrifft biokompatible zwei- oder mehrkomponentige Hydrogele, besonders zur Einbettung und Kultivierung von biologischen Zellen und/oder als pharmazeutische Formulierung sowie neuartige peptidische Vernetzer zur Vernetzung von bindungsfunktionalisierten Polymeren zu solchen Hydrogelen. Die Erfindung stellt dazu ein lineares Molekül bereit, welches eine Molekülmasse von etwa 3 bis etwa 60 kDa besitzt und im Bereich der Molekülenden jeweils eine Bindungsfunktion aufweist und komplementär bindungsfunktionalisierte Polymere vernetzen kann.

Polymerische Hydrogele zur Verwendung als biokompatible oder biomimetische Substrate, insbesondere für die Kultivierung von biologischen Zellen und Geweben, sind bekannt. Zwei- oder mehrkomponentige Gele sind meist aus hochmolekularem Polymer als erste Komponente und niedrigmolekularem Vernetzer als zweite Komponente aufgebaut. Bekannte zwei- oder mehrkomponentige Hydrogele mit peptidischer Vernetzung weisen beispielsweise mehrarmige makromolekulare Strukturen aus Polyethylenglykol (PEG) auf, die über kurze lineare Peptide vernetzt sind. Die Vernetzermoleküle besitzen bekanntermaßen eine lineare Molekülstruktur. Sie weisen mindestens zwei, bevorzugt endständige Bindungsfunktionen eines ersten Typs, zum Beispiel Thiolfunktion, auf, die mit dazu komplementären Bindungsfunktionen eines zweiten Typs, zum Beispiel Maleimidfunktion, der zu vernetzenden Polymere, zum Beispiel PEG, PVA, Albumin oder Dextran, konjugieren und so ein vernetztes Gel bilden.

Solche Hydrogele können ferner so ausgestaltet sein, dass sie durch die Stoffwechselaktivität der darin kultivierten Zellen bioabbaubar oder durch andere zeitabhängige Prozesse spaltbar sind. Die Spaltbarkeit erlaubt, dass die Hydrogel-Matrix im Laufe der Kultivierung durch eine von den Zellen selbst gebildete extrazelluläre Matrix (ECM) ersetzt wird und/oder die Wanderung von Zellen in den Hydrogelen. Um eine Bioabbaubarkeit oder Spaltbarkeit der Hydrogele zu erreichen, können diese bekanntermaßen mit aus linearen Peptiden aufgebauten peptidischen Vernetzern hergestellt werden. Die Peptide sind durch bioaktive Moleküle, insbesondere Enzyme wie Peptidasen oder Proteinasen, beispielsweise Matrixmetalloproteinasen (MMP) spaltbar. Durch die intramolekulare Spaltung des Vernetzers wird die Hydrogelstruktur wieder aufgelöst.

Nachteilig bei bekannten Hydrogelen ist vor allem, dass zur Bildung eines stabilen Hydrogels, das insbesondere für die Zellkultivierung geeignet ist, die Konzentration der Vernetzerpeptide hoch, das heißt in jedem Fall mehr als 10 mmol/L, in der Regel 20 mmol/L oder mehr, sein muss. Bekannte Vernetzerpeptide sind teuer in der Produktion. Außerdem beeinträchtigt die hohe Konzentration des Vernetzers die Möglichkeit der Zugabe weiterer löslicher Komponenten in das Hydrogel bei der Herstellung und reduziert den Wassergehalt des Hydrogels. Solche bekanntermaßen vernetzten Hydrogele sind deshalb verbesserungsbedürftig.

BIOSIS PREV200800472961 erwähnt einen Vernetzer, der aus einem enzymatisch spaltbarer Sequenz und einem kurzkettigen Ethylenoxid-Polymer besteht, sowie auch ein Hydrogel, was mittels dieses Vernetzer herstellbar ist. Verfahren zur Herstellung der obengenannten Vernetzers und Hydrogels sind aus Macromolecular Rapid Communications 27(13), 5. Juli 2006, Ss. 1004-1008 zu vernehmen.

Die Erfindung hat sich zur Aufgabe gestellt, peptidische Vernetzer für die Herstellung von mehrkomponentigen Hydrogelen zu verbessern. Ein der Erfindung zugrunde liegendes technisches Problem besteht darin, Mittel und Verfahren bereitzustellen, wodurch die Hydrogelbildung bei geringeren Vernetzerkonzentrationen erfolgen kann als seither möglich.

Zur vollständigen Lösung des technischen Problems stellt die Erfindung ein neuartiges peptidisches Vernetzermolekül mit im Bereich des oder an dem jeweiligen Molekülende jeweils mindestens einer Bindungsfunktion zur Vernetzung von komplementär bindungsfunktionalisierten Polymeren zur Verwendung als Polymervernetzer für zwei- oder mehrkomponentige Hydrogele bereit. Dieses Vernetzermolekül ist erfindungsgemäß ein hochmolekulares lineares Molekül aus mindestens zwei linear verbundenen und insbesondere kovalent konjugierten, Komponenten, und zwar mindestens einer bevorzugt hochmolekularen Polymerkomponente und mindestens einer, bevorzugt niedermolekularen, Peptidkomponente. Erfindungsgemäß weist das Vernetzermolekül eine Gesamtmolekülmasse von 3 kDa oder mehr, bevorzugt 5 kDa oder mehr, insbesondere von 3 kDa bis 60 kDa oder 5 kDa bis 60 kDa, auf.

Im Zusammenhang mit der Erfindung wird unter einem "zwei- oder mehrkomponentigen Hydrogel" eine aus Vernetzermolekülkomponenten und davon verschiedenen hochmolekularen Polymerkomponenten aufgebaute Struktur verstanden, worin die Polymere über die Vernetzermoleküle miteinander zu einem Gel vernetzt sind. Die Vernetzung findet bevorzugt über kovalente Konjugationsreaktionen mit jeweils komplementären Bindungsfunktionen der Vernetzermoleküle und der zu vernetzenden Polymermoleküle statt.

Die Erfindung stellt also ein lineares hochmolekulares Vernetzermolekül für zwei- oder mehrkomponentige Hydrogele bereit, worin der Abstand der Bindungsfunktionen, die im Bereich des oder am jeweiligen Ende des Moleküls lokalisiert sind, gegenüber bekannten Vernetzerpeptiden deutlich vergrößert ist. Die Molekülmasse des linearen Moleküls dient als Maß für den Abstand der im Wesentlichen endständigen Bindungsfunktionen.

Die Erfinder fanden überraschend, dass durch die Verwendung des erfindungsgemäß hochmolekularen Polymervernetzers die zur Bildung eines zwei- oder mehrkomponentigen Hydrogels notwendige Konzentration des Vernetzers gegenüber bekannten peptidischen Polymervernetzern deutlich reduziert werden kann. Vorteilhafterweise ist die zur Gelbildung erforderliche Konzentration gegenüber den aus dem Stand der Technik bekannten peptidischen Polymervernetzern mindestens um den Faktor 2, bevorzugt mindestens um den Faktor 3, besonders bevorzugt mindestens um den Faktor 10, reduziert. Ein erfindungsgemäßer Polymervernetzer bildet bereits bei Konzentrationen der Bindungsfunktionen von etwa 3 mmol/L, bezogen auf das Hydrogel, stabile Hydrogele. Daneben wurde gefunden, dass die Effizienz der Vernetzung auch im Bezug auf die Geschwindigkeit der Vernetzungsreaktion verbessert ist.

Ohne an die Theorie gebunden sein zu wollen, macht sich die Erfindung die Erkenntnis zunutze, dass die Häufigkeit des Ereignisses, dass der Vernetzer mit demselben Polymer mehrfach bindet und so nicht mehr zur Vernetzung mit einem anderen Polymermolekül zur Verfügung steht, mit zunehmendem molekularen Abstand der an der Vernetzung beteiligten Bindungsfunktionen im Vernetzermolekül abnimmt. Durch das erfindungsgemäß hochmolekulare Vernetzermolekül sind die Bindungsfunktionen darin derart beabstandet, dass in einer vergleichsweise weitaus größeren Anzahl der Fälle ein Vernetzermolekül zumindest zwei verschiedene Polymermoleküle vernetzt und somit zur Vernetzung der Polymermoleküle zur Bildung des Gels beiträgt.

Die reduzierte Konzentration des Vernetzers erlaubt vorteilhafterweise, Hydrogele mit einem höheren Wassergehalt bereitzustellen als es mit bekannten Vernetzern, besonders mit bekannten peptidischen Vernetzern, möglich war. Alternativ oder zusätzlich gelingt es, in den Hydrogelen weitere lösliche Bestandteile, besonders Medienkomponenten für die Zellkultur und/oder anderen Reagenzien, in anderer Zusammensetzung und/oder anderer, bevorzugt höherer, Konzentration einzubringen, was in bekannten Gelen nachteiligerweise durch die dort herrschende hohe Konzentration an herkömmlichen Vernetzern nicht möglich ist.

Die Erfindung stellt dabei auch eine neuartige Lehre bereit, solche erfindungsgemäß vorteilhaften hochmolekularen Vernetzer auf einfache und damit wirtschaftliche Weise zu erhalten. Um den erfindungsgemäß hochmolekularen Vernetzer zu erzeugen, sieht die Erfindung vor, eine im Wesentlichen kurzkettige, das heißt niedermolekulare Peptidkomponente mit einer hochmolekularen Polymerkomponente intramolekular zu verknüpfen. Die Erfindung vermeidet vorteilhafterweise vollständig die bekanntermaßen aufwändige Herstellung eines längerkettigen Peptids, um die erfindungsgemäß hohe Molekularität des Vernetzers und damit die günstige große Beabstandung der Bindungsfunktionen im Bereich der Molekülenden zu erreichen. Die Erfindung sieht an dessen Stelle den Einbau einer oder mehrerer bevorzugt hochmolekularer Polymerkomponenten in das Vernetzermolekül vor.

Vorteilhafterweise kann der erfindungsgemäße Vernetzer bekannte Vernetzermoleküle mit bekannten Bindungsfunktionalisierungen ersetzen, sodass die jeweils etablierten Vernetzungsreaktionen zur Herstellung zwei- oder mehrkomponentiger Hydrogele mit peptidischer Vernetzung weiterhin in an sich bekannter Weise durchgeführt werden können. Es bedarf keiner weiteren Anpassung der Verknüpfungschemie, und es werden zumindest gleichwertige Hydrogele wie mit bisher bekannten Vernetzermolekülen erhalten.

Die Erfindung betrifft auch die Verwendung in Vernetzerzusammensetzungen zusammen mit herkömmlichen Polymervernetzern. Darin sind die erfindungsgemäßen Vernetzermoleküle in Anteilen enthalten, die hier dargestellte vorteilhafte Wirkungen in der Vernetzerzusammensetzungen spürbar werden lassen. Je nach gewünschter Stärke des Effekts, kann der Fachmann den Anteil der Vernetzermoleküle der Vernetzerzusammensetzung ohne weiteres bestimmen. Eine Variante der Erfindung stellt Vemetzerzusammensetzungen bereit, worin erfindungsgemäße Vernetzermoleküle von 10 bis 50%, bezogen auf den molaren Anteil, enthalten ist.

Im Zusammenhang mit der Erfindung wird unter einer Polymerkomponente des Vernetzermoleküls" ein bevorzugt hochmolekulares, bevorzugt lineares unverzweigtes Molekül verstanden, das aus einfach niedermolekularen Monomeren aufgebaut ist. Davon zu unterscheiden ist die "Peptidkomponente des Vernetzermoleküls", die ein, bevorzugt niedermolekulares, Polyaminosäuremolekül ist, das eine Abfolge von bevorzugt unterschiedlichen Aminosäuren aufweist.

In bevorzugter Ausgestaltung der Erfindung weist die Polymerkomponente des Polymervernetzers eine Molekülmasse auf, die im Wesentlichen zumindest der Molekülmasse der Peptidkomponente entspricht oder größer ist; das Verhältnis der Molekülmasse der Peptidkomponente zu der Molekülmasse des Polymervernetzers beträgt von 1:1 bis etwa 1:20 oder mehr, bevorzugt 1:5 bis 1:10 oder 1:5 bis 1:20. Das Polymermolekül des Vernetzers besitzt bevorzugt eine Molekülmasse, die im Vergleich zu der Molekülmasse des Peptidmolekül des Vernetzers um zumindest den Faktor 2, bevorzugt um Faktor 5 oder mehr, besonders bevorzugt um Faktor 10 oder mehr höher ist. Das, bevorzugt hochmolekulare, Polymermolekül des Vernetzers weist dabei bevorzugt eine Molekülmasse von mindestens 3 kDa, 4 kDa oder mehr und bevorzugt 10 kDa oder mehr auf. In einer Variante beträgt die Molekülmasse von 3 bis etwa 50 kDa, in einer weiteren Variante von 5 bis 25 kDa. Das Peptidmolekül des Vernetzers weist bevorzugt eine Molekülmasse von 5 kDa oder weniger, bevorzugt 3 kDa oder weniger, mehr bevorzugt 2 kDa oder weniger auf. In einer Variante beträgt die Molekülmasse des Peptids von 300 Da (0,3 kDa) bis 5 kDa, in einer weiteren Variante von 500 Da (0,5 kDa) bis 2 kDa. In einer besonderen Ausgestaltung trägt die Peptidkomponente nur wenige Aminosäuren, besonders 3 oder 4 oder 5 oder mehr.

Im Zusammenhang mit der Erfindung wird die Molekülmasse, dargestellt in der Einheit Da (Dalton), als Äquivalenzmaß für die Größe oder Länge der Moleküle verwendet. Es versteht sich, dass 1 Da an sich der Molekülmasse 1 u entspricht.

Bevorzugt sind die mindestens eine Polymerkomponente und die mindestens eine Peptidkomponente in dem Vernetzermolekül intramolekular durch kovalent konjugierende Bindungsfunktionen-Paare verknüpft. Daher ist jeweils mindestens eines der Molekülenden der Polymer- und der Peptidkomponente bindungsfunktionalisiert, um mit einer komplementären Bindungsfunktion der jeweils anderen Komponente eine kovalente Konjugation einzugehen. Es versteht sich, dass mindestens eine der an dem Molekül vorgesehenen Bindungsfunktionen durch eine entsprechende Schutzgruppe geschützt werden kann, um die Selbstkonjugation der Moleküle zu unterbinden.

Bevorzugt ist eine Bindungsfunktion zur intramolekularen Verknüpfung eine nukleophile Gruppe. Die dazu komplementäre Bindungsfunktion ist dann eine elektrophile Funktion, besonders eine elektrophile Doppelbindung. Bevorzugt sind Reaktionen. die funktionelle Gruppen der Peptidkomponente, an Seitenketten, am N- und C-Terminus, unberührt lassen. Die Konjugation erfolgt dabei über eine Addition. Es zeigt sich, dass solche Additionsreaktionen vorteilhafterweise keine Abgangsgruppen produzieren. Zusätzliche Waschschritte zur Beseitigung von bei Verknüpfungsreaktionen auftretenden Reaktionspartnern und Abgangsgruppen aus dem gebildeten Konjugat können vermieden werden. Die intramolekulare Konjugation kann daher vorteilhafterweise kontaminationsfrei physiologische Bedingungen für die Kultivierung biologischen Zellen erhalten. Bevorzugte Reaktionspartner sind Thiole, beispielsweise Thiolstrukturen in Aminosäuren (zum Beispiel Cystein) der Peptidkomponente, die an Doppelbindungen von Maleimiden, Vinylsulfon, Acrylaten, bevorzugt Acrylamid, Metacrylat und Acrylat, oder entsprechende Verbindungen koppeln.

Eine bevorzugte Reaktion zur Bildung der intramolekularen Verknüpfung der Komponenten des Vernetzers ist die Michael-Addition, die bevorzugt auf Basis von einem oder mehreren Thiol-Gruppen als erster Reaktionspartner und bevorzugt Maleimid, Vinylsulfon oder Acrylaten als zweiter komplementärer Reaktionspartner durchgeführt werden.

Alternative Reaktionstypen für die intramolekulare Verknüpfung der Komponenten des Vernetzermoleküls sind Substitution, chemoselektive Ligation, reduktive Aminierung, die Staudinger-Ligation und die sogenannte Klick-Chemie. Bei der Substitution treten als Reaktionspartner bevorzugt Amine auf, die mit Carboxyl- oder Hydroxyl-Gruppen reagieren. Unter Gegenwart von Reduktionsmitteln werden die Reaktionspartner Aldehyd- und Amino-Gruppen verknüpft. Bei der chemoselektiven Ligation treten als Reaktionspartner bevorzugt Thiol und Bromacetyl oder Aldehyd und Aminoxy-Gruppen auf. Bei der Staudinger-Ligation werden bevorzugt Phosphin-Gruppen mit Azid-Gruppen verknüpft. Bei der sogenannten Klick-Chemie werden Alkyn- oder Cyclooctin-Gruppen mit Azid-Gruppen, gegebenenfalls in Gegenwart von Kupfer, verknüpft. Weitere einsetzbare Bindungsfunktionen zur intramolekularen Verknüpfung der erfindungsgemäßen Vernetzerkomponenten sind die Bindungspartner Streptavidin/Avidin und Biotin oder entsprechende Bindungsfunktionen. Die Erfindung ist in Bezug auf die intramolekularen Bindungsfunktionen nicht auf die vorgenannten beschränkt. Der Fachmann kennt diese und weitere analoge Bindungsfunktionen, die er entsprechend dem Anwendungsgebiet des herzustellenden Vernetzermoleküls in an sich bekannter Weise einsetzen kann.

In einer Ausgestaltung sind die Komponenten im erfindungsgemäßen Vernetzermolekül über eine Thiol-Maleimid-Konjugation verbunden. In einer ersten Variante davon trägt die Polymerkomponente im Bereich jedes Molekülendes eine Maleimidfunktion und die Peptidkomponente des Vernetzers trägt im Bereich des Molekülendes eine Thiolfunktion und im Bereich des anderen Molekülendes eine Maleimidfunktion. In einer alternativen Variante trägt die Polymerkomponente jeweils die Thiolfunktion, und die Peptidkomponente trägt jeweils eine Thiolfunktion und eine Maleimidfunktion. Die Thiolfunktion der Peptidkomponente wird bevorzugt durch einen Cysteinrest realisiert. Der Cysteinrest ist in einer ersten Variante C-terminal lokalisiert. In einer alternativen Variante ist der Cysteinrest am N-terminalen Ende lokalisiert. Als Schutzgruppe der Thiolfunktion kann eine Tert-Butylgruppe vorhanden sein, die zur Bildung der intramolekularen Verknüpfung von Peptid- und Polymerkomponente entfernt wird.

In dieser bevorzugten Ausgestaltung tragen die Komponenten des Vernetzermoleküls zur intramolekularen Verknüpfung, dieselben Bindungsfunktionen oder analoge Bindungsfunktionen, wie sie auch an den jeweiligen Molekülenden des Vernetzermoleküls vorgesehen sind und bei der Verwendung des Vernetzers zur Vernetzung bindungsfunktionalisierter Polymere dienen können. Dies erlaubt vorteilhafterweise die einfachere Herstellung, da insgesamt eine geringe Zahl an unterschiedlichen Bindungsfunktionen realisiert werden muss. Die Herstellung solcher Vernetzermoleküle ist nachstehend näher erläutert.

In einer bevorzugten Variante der Erfindung ist als Verknüpfung des Vernetzers mit dem Polymer zur Bildung des Hydrogels die radikalische Verknüpfung ausgeschlossen, das heißt es findet zur Bildung des Hydrogels durch den erfindungsgemäßen Polymervernetzter keine radikalische Verknüpfung statt. Die Erfindung betrifft in bevorzugter Ausgestaltung nicht die Verwendung des Vernetzermoleküls zur Herstellung radikalisch verknüpfter einkomponentiger Gele.

In einer anderen bevorzugten Ausgestaltung tragen die Komponenten des Vernetzermoleküls zur intramolekularen Verknüpfung andere Bindungsfunktionen, die mit den an den jeweiligen Molekülenden des Vernetzermoleküls vorgesehenen Bindungsfunktionen, die bei der Verwendung des Vernetzers zur Vernetzung bindungsfunktionalisierter Polymere dienen können, nicht übereinstimmen oder mit diesen komplementär sind, um eine Verknüpfung einzugehen. Auch diese Variante ist einer einfachen Herstellung zugänglich. Insbesondere kann auf Schutzgruppen an Bindungsfunktionen zur Verhinderung von Selbstkonjugation verzichtet werden. Die Herstellung solcher Vernetzermoleküle ist ebenfalls nachstehend erläutert.

In einer Ausgestaltung der Erfindung weist der erfindungsgemäße Vernetzer mindestens eine spaltbare intramolekulare Verknüpfung auf. Die Erfindung sieht vor, dass die mindestens eine spaltbare intramolekulare Verbindung in dem Vernetzer zumindest zwischen den jeweils im Bereich der Molekülenden lokalisierten Bindungsfunktionen des Vernetzers lokalisiert ist. Dadurch kann durch Spaltung des Vernetzers eine Auflösung der Vernetzung der Polymere im Hydrogel und damit eine Verflüssigung des Hydrogels erreicht werden. In einer ersten Variante dieser Ausführung ist die spaltbare Verknüpfung an der Verbindungsstelle der mindestens einen Peptidkomponente und der erfindungsgemäß vorgesehenen hochmolekularen Polymerkomponente realisiert. In einer alternativen bevorzugten Variante ist die spaltbare Verknüpfung innerhalb mindestens einer Peptidkomponente des Vernetzers realisiert. Durch solche spaltbaren Verbindungen kann das mit dem erfindungsgemäßen Vernetzer gebildete Hydrogel durch gezielte Einwirkung wieder verflüssigt werden. Eine solche gezielte Einwirkung zur Verflüssigung kann durch außen (zum Beispiel Temperatur, Strahlung) und alternativ oder zusätzlich durch Wirkung von in dem Hydrogel kultivierten biologischen Zellen (zum Beispiel Enzymwirkung) ausgehen.

In einer ersten Ausgestaltung ist die spaltbare intramolekulare Verbindung eine wasserspaltbare Verbindung, die durch den Einfluss von und durch die Umsetzung mit Wasser gespalten werden kann. Alternativ oder zusätzlich ist die spaltbare Verbindung eine mittels der Aktivität eines Katalysators spaltbare Verbindung. Ein bevorzugter Katalysator ist ein biologisches Enzym. Bevorzugte biologische Enzyme sind Peptidasen und Proteinasen oder allgemein Hydrolasen, die eine Hydrolyse der spaltbaren Verbindung katalysieren.

In einer alternativen oder zusätzlichen Ausgestaltung ist die spaltbare intramolekulare Verbindung eine von dem pH-Wert der Umgebung abhängige Verknüpfung, die durch Änderung des pH-Werts gespalten werden kann. In einer weiteren Ausgestaltung ist die spaltbare intramolekulare Verbindung, alternativ oder zusätzlich, eine temperaturabhängige Verknüpfung, die insbesondere durch Temperaturerhöhung spaltbar ist. Daneben sind als spaltbare intramolekulare Verbindungen andere durch Energieeintrag, besonders elektromagnetische Strahlung, ausgewählt aus Mikrowellen, Lichteinwirkung, UV- und/oder IR-Strahlung, spaltbare Verknüpfungen in dem Vernetzermolekül, vorgesehen.

In einer bevorzugten Ausgestaltung weist die Peptidkomponente des Polymervernetzers zumindest eine bioaktive Aminosäureabfolge auf.

Unter einer "bioaktive" Aminosäureabfolge wird eine Aminosäuresequenz oder ein Aminosäuremotiv verstanden, das von biologischen Systemen, beispielsweise lebenden biologischen Zellen, "erkannt" wird, wobei mindestens eine Komponente der biologischen Zelle mit der bioaktiven Aminosäureabfolge in Wechselwirkung tritt. Eine solche Komponente kann ein enzymatisch aktives Protein sein. Besonders bevorzugt ist die erfindungsgemäße bioaktive Aminosäureabfolge durch Peptidase-/Proteinaseaktivität spaltbar. Demgemäß weist die Peptidkomponente des erfindungsgemäßen Konjugats zumindest eine biospaltbare, das heißt enzymatisch spaltbare Aminosäureabfolge auf.

Unter Matrixmetalloproteinasen werden im Zusammenhang mit der vorliegenden Erfindung insbesondere Kollagenasen, Gelatinasen, Stromelysine, Matrilysine, Metalloelastasen und Membran-Metalloproteinasen verstanden. Diese binden an sogenannte "Proteinspaltungsstellen" mit vom Typ der Metalloproteinase abhängiger Spezifität. Solche Aminosäuresequenzen sind an sich bekannt (Nagase und Fields, Biopolymers (Peptid Science), 40 (1996): 399-416).

Die Tabellen 1 und 2 listen bevorzugte Sequenzmotive/Aminosäureabfolgen der erfindungsgemäß bevorzugt spaltbar ausgeführten Peptidvernetzer auf. Die Tabelle 2ABCDEF zeigt Sequenzmotive, geordnet nach den jeweils spaltenden Metalloproteinasen (MPP1 MPP10). Es versteht sich, dass die Erfindung nicht auf diese konkreten Sequenzen beschränkt ist und auch funktionelle Abwandlungen davon umfasst.

**Tabelle 1:**

| |
|---|
| Ac-GCRD-GPQG / IWGQ-DRCG-NH2 |
| Ac-GCRD-GPQG / IAGQ-DRCG-NH2 |
| Ac-GCRD-GDQGIAGF-DRCG-NH2 |
| Ac-GCYK / NRD-CG |
| NH2-GGGLGPAGGK-NH2 |
| NH2-GGCLGPACGK-NH2 |
| Dnp-PLGLWA-(D)Arg-NH2 |
| Mca-PLGL-Dpn-(D)Arg-NH2 |
| Mca-KPLGL-Dpa-AR-NH2 |

**Tabelle 2:**

| **A. MMP1∼MMP8** | |
|---|---|
| Human type I collagen (al) | Ala-Pro-Gln-Gly₇₇₅∼Ile₇₇₆-Ala-Gly-Gln |
| Human type I collagen (a2) | Gly-Pro-Gln-Gly₇₇₅∼Leu₇₇₆-Leu-Gly-Ala |
| Human type I1 collagen | Gly-Pro-Gln-Gly₇₇₅∼Leu₇₇₆-Ala-Gly-Gln |
| Human type 111 collagen | Gly-Pro-Leu- Gly₇₇₅∼Ile₇₇₆-Ala-Gly-Ile |
| Human a2-macroglobulin | Gly-Pro-Glu- Gly₆₇₉∼Leu₆₈₀-Arg-Val-Gly |
| Rat a,-macroglobulin | Ala-Ala-Tyr-His₆₈₁∼Leu₆₈₂-Val-Ser-Gln |
| Rat a2-macroglobulin | Met-Asp-Ala-Phe691~Leu692-Glu-Ser- Ser |
| Rat a I -macroglobulin | Glu-Pro-Gln-Ala₆₈₃∼Leu₆₈₄-Ala-Met-Ser |
| Rat a,-macroglobulin | Gln-Ala-Leu-Ala₆₈₅∼Met₆₈₆-Ser-Ala-Ik! |
| Chicken ovostatin | Pro-Ser-Tyr-Phe673~Leu674-Asn-Ala- G1y |
| Human pregnancy zone protein | Tyr-Glu-Ala-Gly₆₈₅∼Leu₆₈₆-Gly-Val-Val |
| Human pregnancy zone protein | Ala-Gly-Leu-Gly₆₈₇∼Val₆₈₈-Val-Glu-Arg |
| Human pregnancy zone protein | Ala-Gly-Leu-Gly₇₅₇∼Ile₇₅₈-Ser-Ser-Thr |
| a,-Protease inhibitor | Gly-Ala-Met-Phe₃₅₂∼Leu₃₅₃-Glu-Ala-Ile |
| Human aggrecan | Ile-Pro-Glu-Asn₃₄₁∼Phe₃₄₂-Phe-Gly-Val |
| Human aggrecan | Thr-Glu-Gly-Glu₃₇₃∼Ala₃₇₄-Arg-Gly-Ser |
| Human cartilage link | Arg-Ala-Ile-His₁₆∼Ile₁₇-Gln-Ala-Glu |
| Human insulin-like growth factor binding protein-3 | Leu-Arg-Ala-Tyr₉₉∼Leu₁₀₀-Leu-Pro-Ala |
| | |

| **B. MMP2** | |
|---|---|
| Guinea pig a 1(I) gelatin | Gly-Ala-Hyp-Gly₅₄₇∼Leu₅₄₈-Glx-Gly-His |
| Rat al(I) gelatin | Gly-Pro-Gln-Gly₁₉₀∼Val₁₉₁-Arg-Gly-Glu |
| Rat a 1 (I) gelatin | Gly-Pro-Ala-Gly₂₇₇∼Va1₂₇₈-Gln-Gly-Pro |
| Rat a 1 (I) gelatin | Gly-Pro-Ser-Gly₃₀₁∼Leu₃₀₂-Hyp-Gly-Pro |
| Rat a I(1) gelatin | Gly-Pro-Ala-Gly₃₃₁∼Glu₃₃₂-Arg-Gly-Ser |
| Rat a I (I) gelatin | Gly-Ala-Lys-Gly₃₆₁∼Leu₃₆₂-Thr-Gly-Ser |
| Rat a 1 (I) gelatin | Gly-Pro-Ala-Gly₃₈₂∼Gln₃₈₃-Asp-Gly-Pro |
| Rat a 1 (I) gelatin | Gly-Pro-Ala-Gly₆₃₄∼Phe₆₃₅-Ala-Gly-Pro |
| Rat al(1)gelatin | Gly-Pro-Ile-Gly₆₇₆∼Asn₆₇₇-Val-Gly-Ala |
| Rat a 1 (I) gelatin | Gly-Pro-Hyl-Gly₆₈₅∼Ser₆₈₆-Arg-Gly-Ala |
| Bovine type I collagen (a 1) | Gly-Pro-Gln-Gly₇₇₅∼Ile₇₇₆-Ala-Gly-Gln |
| Bovine type I collagen (a2) | Gly-Pro-Gln-Gly₇₇₅∼Leu₇₇₆-Leu-Gly-Ala |
| Human aggrecan | Ile-Pro-Glu- Asn341~-Phe342-Phe-Gly- Val |
| Human galectin-3 | Pro-Pro-Gly-Ala₆₂∼Tyr₆₃-His-Gly-Ala |
| Human cartilage link | Arg-Ala-Ile-His₁₆∼Ile₁₇-Gln-Ala-Glu |
| Human cartilage link | Gly-Pro-His-Leu₂₅∼Leu₂₆-Val-Glu-Ala |
| Human insulin-like growth factor binding protein-3 | Leu-Arg-Ala-Tyr₉₉∼Leu₁₀₀-Leu-Pro-Ala |
| | |

| **C. MMP3** | |
|---|---|
| Human a2-macroglobulin | Gly-Pro-Glu-Gly₆₇₉∼Leu₆₈₀-Arg-Val-Gly |
| Human a2-macroglobulin | Arg-Val-Gly-Phe₆₈₄∼Tyr₆₈₅-Glu-Ser-Asp |
| Human a,-antichymotrypsin | Leu-Leu-Ser-Ala₃₆₀∼Leu₃₆₁-Val-Glu-Thr |
| a,-protease inhibitor | Glu-Ala-Ile-Pro₃₅₇Met₃₅₈-Ser-Ile-Pro |
| Antithrombin 111 | Ile-Ala-Gly-Arg₃₈₅∼Ser₃₈₆-Leu-Asn-Pro |
| Chicken ovostatin | Leu-Asn-Ala-Gly₆₇₇∼Phe₆₇₈-Thr-Ala-Ser |
| Human aggrecan | Ile-Pro-Glu-Asn₃₄₁∼Phe₃₄₂-Phe-Gly-Val |
| Substance P | Lys-Pro-Gln-Gln₆∼Phe₇-Phe-Gly-Leu |
| Human ProMMP-1 | Asp-Val-Ala-Gln₈₀∼Phe₈₁-Val-Leu-Thr |
| Human ProMMP-3 | Asp-Thr-Leu-Gly₆₈∼Val₆₉-Met-Arg-Lys |
| Human ProMMP-3 | Asp-Val-Gly-His₈₂∼Phe₈₃-Arg-Thr-Phe |
| Human ProMMP-8 | Asp-Ser-Gly-Gly₇₈∼Phe₇₉-Met-Leu-Thr |
| Human ProMMP-9 | Arg-Val-Ala-Glu₄₀∼Met₄₁-Arg-Gly-Glu |
| Human ProMMP-9 | Asp-Leu-Gly-Arg₈₇∼Phe₈₈-Gln-Thr-Phe |
| Human fibronectin | Pro-Phe-Ser-Pro₆₈₉∼Leu₆₉₀-Val-Ala-Thr |
| Human insulin-like growth factor binding protein-3 | Leu-Arg-Ala-Tyr₉₉∼Leu₁₀₀-Leu-Pro-Ala |
| | Ala-Pro-Gly-Asn₁₀₉∼Ala₁₁₀-Ser-Glu-Ser |
| | Phe-Ser-Ser-Glu₁₇₆∼Ser₁₇₇-Lys-Arg-Glu |
| Bovine a1 (11) collagen, N-telopeptide | Ala-Gly-Gly-Ala₁₁₅∼Gln₁₁₆-Met-Gly-Val |
| Bovine a I(I1) collagen, N-telopeptide | Gln-Met-Gly-Val₁₁₉∼Met₁₂₀-Gln-Gly-Pro |
| Bovine a 1 (IX) collagen, NC2 | Met-Ala-Ala-Ser∼Leu-Lys-Arg-Pro |
| Bovine a2(IX) collagen, NC2 | ∼Ala-Lys-Arg-Glu |
| Bovine a3(IX) collagen, NC2 | ∼Leu-Arg-Lys-Pro |
| Bovine a 1 (XI) collagen, N-telopeptide | Gln-Ala-Gln-Ala-Ile-Leu-Gln-Gln |
| Human cartilage link | Arg-Ala-Ile-His₁₆∼Ile₁₇-Gln-Ala-Glu |
| Bovine insulin, B chain | Leu-Val-Glu-Ala₁₄∼Leu₁₅-Tyr-Leu-Val |
| Bovine insulin, B chain | Glu-Ala-Leu-Tyr₁₆∼Leu₁₇-Val-Cys-Gly |
| | |

| **D. MMP7** | |
|---|---|
| Human aggrecan | Ile-Pro-Glu-Asn₃₄₁∼Phe₃₄₂-Phe-Gly-Val |
| Human cartilage link | Gly-Pro-His-Leu₂₅∼Leu₂₆-Val-Glu-Ala |
| Human prourokinase | Pro-Pro-Glu-Glu₁₄₃∼Leu₁₄₄-Lys-Phe-Gln |
| | |

| **E. MMP9** | |
|---|---|
| Human type V collagen (al) | Gly-Pro-Pro-Gly₄₃₉∼Val₄₄₀-Val-Gly-Pro |
| Human type V collagen (a2) | Gly-Pro-Pro-Gly₄₄₅∼Leu₄₄₆-Arg-Gly-Glu |
| Human type XI collagen (al) | Gly-Pro-Gly-Gly₄₃₉∼Val₄₄₀-Val-Gly-Pro |
| Human aggrecan | Ile-Pro-Glu-Asn₃₄₁∼Phe₃₄₂-Phe-Gly-Val |
| Human galectin-3 | Pro-Pro-Gly-Ala₆₂∼Tyr₆₃-His-Gly-Ala |
| Human cartilage link | Arg-Ala-Ile-His₁₆∼Ile₁₇-Gln-Ala-Glu |
| | |

| **F. MMP10** | |
|---|---|
| Human cartilage link | Arg-Ala-Ile-His₁₆∼Ile₁₇-Gln-Ala-Glu |
| Human cartilage link | Gly-Pro-His-Leu₂₅∼Leu₂₆-Val-Glu-Ala |

In einer alternativen oder bevorzugt zusätzlichen Ausgestaltung ist die bioaktive Aminosäureabfolge eine Signalsequenz, die eine biologische Reaktion auslöst, insbesondere Bestandteil einer Signalkaskade einer biologischen Zelle. In einer alternativen oder bevorzugt zusätzlichen Ausgestaltung ist die bioaktive Aminosäureabfolge eine spezifische Bindesequenz.

Als das Peptidmolekül des erfindungsgemäßen Vernetzers kommen an sich bekannte und in an sich bekannter Weise herstellbare Oligopeptide in Betracht. Solche Oligopeptide sind vorzugsweise synthetisch hergestellt. Bevorzugt ist dabei das Verfahren der Festphasenpeptidsynthese. Im Rahmen der Festphasenpeptidsynthese oder im Anschluss daran kann die Bindungsfunktionalisierung des Peptids erfolgen. Eine Variante der konkreten Umsetzung ist in den nachfolgenden Erläuterungen und Beispielen aufgeführt. In einer bevorzugten Ausgestaltung gliedert sich die Bereitstellung des bindungsfunktionalisierten Peptids in zumindest folgende Teilschritte auf: In einem ersten Schritt wird ein Polyaminosäuremolekül mit C-terminaler Bindungsfunktion, bevorzugt durch Festphasenpeptidsynthese de novo synthetisiert. In einem weiteren Schritt wird das N-terminale Ende des synthetisierten Polyaminosäuremoleküls mit einer dazu komplementären Bindungsfunktion funktionalisiert. Es versteht sich, dass zur Unterdrückung von Selbstkonjugation Schutzgruppen vorgesehen sein können.

Als das hochmolekulare lineare Polymermolekül des erfindungsgemäßen Vernetzers kommen an sich bekannte hochmolekulare Polymere zum Einsatz. Diese sind bevorzugt ausgewählt aus linearen Polymeren, die im Bereich deren jeweiligen Molekülenden mit Bindungsfunktionen funktionalisierbare Reste aufweisen, insbesondere eine Hydroxylgruppe. Bevorzugt ist die Polymerkomponente ausgewählt aus der Gruppe bestehend aus: Polyethylenglykolen (PEG), Polypropylenglykolen (PPG) und entsprechenden Polymeren sowie Block-Copolymere aus mehreren davon, wie Block-Copolymere aus PPG und PEG. Als Polymerkomponente des Vernetzers kommen auch Mischungen von zwei oder mehreren dieser Polymere in Betracht. Eine Ausgestaltung der hochmolekularen Polymerkomponente ist lineares PEG, das endständig gleichartig funktionalisiert ist, besonders Dithiol-PEG beziehungsweise Dimaleimid-PEG.

Zur Vernetzung mit dem bindungsfunktionalisiertem Polymer weist der Vernetzer mindestens zwei Bindungsfunktionen, und zwar jeweils mindestens eine, im Bereich der beiden gegenüberliegenden Molekülenden des Vernetzers auf. Besonders bevorzugt ist die Bindungsfunktion endständig. In einer alternativen Ausgestaltung liegt die Bindungsfunktion in der unmittelbaren Umgebung des Molekülendes, insbesondere eine, zwei, drei, vier oder fünf Monomereinheiten vor dem Molekülende. Beispielsweise weist das Vernetzermolekül zu zumindest einem Molekülende hin die Peptidkomponente auf. In dieser Peptidkomponente ist bevorzugt die endständige Monomereinheit, das heißt die endständige Aminosäure, bindungsfunktionalisiert; alternativ bevorzugt ist die zweite, dritte, vierte, fünfte oder sechste Aminosäure, vom Molekülende an gezählt, bindungsfunktionalisiert.

In einer Variante befinden sich an dem Molekülende des Vernetzers oder im Bereich des Molekülendes zwei oder mehr Bindungsfunktionen zur Verknüpfung mit jeweils komplementär mehrfach bindungsfunktionalisierten Polymeren. In einer bevorzugten Variante trägt der Vernetzer jeweils genau eine Bindungsfunktion an den beiden Molekülenden.

Bindungsfunktionen eines Polymervernetzers, die mit den komplementären Bindungsfunktionen des zu vernetzenden Polymers wechselwirken und mit diesen bevorzugt kovalent konjugieren, sind an sich bekannt. In Frage kommen, je nach Ausgestaltung des zu vernetzenden Polymers, bevorzugt die vorstehend charakterisierten Bindungsfunktionen, die erfindungsgemäß für die intramolekulare Verknüpfung der Komponenten des Vernetzers einsetzbar sind. Bevorzugt ist eine kovalente Verknüpfung, die sogenannte Konjugation, einer Bindungsfunktion des Vernetzers mit einer komplementären Bindungsfunktion des zu vernetzenden Polymers.

Bei der Vernetzung zur Bildung der Hydrogele ist vorgesehen, dass die zu vernetzenden Polymermoleküle jeweils zumindest drei oder mehr komplementäre Bindungsfunktionen aufweisen. Weisen allgemein die erfindungsgemäßen Vernetzer jeweils mindestens zwei Bindungsfunktionen pro Molekül auf, so sollen die zu vernetzenden Polymere mindestens zwei komplementäre Bindungsfunktionen pro Molekül aufweisen, um die Gelbildung durch Vernetzung einer Vielzahl von Polymeren zu ermöglichen. Es gilt weiter, dass der eine Vernetzungspartner n Bindungsfunktionen pro Molekül und der andere Vernetzungspartner m Bindungsfunktionen pro Molekül aufweist, wobei n+m mindestens 5 beträgt.

Die Erfindung betrifft auch die Herstellung des erfindungsgemäßen hochmolekularen Polymervernetzers. Es wird ein Vernetzer hergestellt, der Bindungsfunktionen eines ersten Typs (A) trägt, worin die erste Komponente des Vernetzers, ausgewählt aus Polymerkomponente und Peptidkomponente, im Bereich der Molekülenden der Komponente jeweils mit der Bindungsfunktion des ersten Typs (A) funktionalisiert ist, und die jeweils andere Komponente des Vernetzers, ausgewählt aus Peptidkomponente und Polymerkomponente, jeweils im Bereich des einen Molekülendes dieser anderen Komponente mit der Bindungsfunktion des ersten Typs (A) und im Bereich des anderen Molekülendes dieser anderen Komponente mit einer Bindungsfunktion eines zweiten Typs (B) funktionalisiert ist, wobei die Bindungsfunktion des zweiten Typs (B) zu der Bindungsfunktion des ersten Typs (A) komplementär ist und mit dieser eine bevorzugt kovalente Konjugation eingeht.

Zur Herstellung eines derartigen Venetzers mit jeweils einer im Bereich von dessen beiden Molekülenden lokalisierten Bindungsfunktion eines ersten Typs (A), der geeignet ist zur Vernetzung von bindungsfunktionalisierten Polymermolekülen, die zu der Bindungsfunktion des Typs (A) komplementäre Bindungsfunktionen des Typs (B) aufweisen. Dazu ist ein Verfahren vorgesehen, worin (i.) eine erste Komponente des Polymervernetzers, also entweder die Polymerkomponente oder Peptidkomponente, bereitgestellt wird, die zumindest im Bereich eines Molekülendes zumindest eine Bindungsfunktion des ersten Typs (A) zur Vernetzungsreaktion zur Bildung des Hydrogels aufweist und im Bereich des anderen Molekülendes zumindest eine Bindungsfunktion eines anderen Typs oder desselben Typs (A) zur intramolekularen Verknüpfung der ersten Komponente mit der anderen, zweiten Komponente des Polymervernetzers, also entweder Peptidkomponente oder Polymerkomponente, aufweist. Es wird (ii.) die andere, zweite Komponente des Polymervernetzers, also entweder Peptidkomponente oder Polymerkomponente, bereitgestellt, die zumindest im Bereich eines Molekülendes oder beider Molekülenden zumindest eine Bindungsfunktion des anderen Typs oder desselben Typs (A) zur intramolekularen Verknüpfung der zweiten Komponente mit der ersten Komponente des Polymervernetzers aufweist und gegebenenfalls im Bereich des anderen Molekülendes zumindest eine Bindungsfunktion des ersten Typs (A) zur Vernetzungsreaktion zur Bildung des Hydrogels aufweist. Es werden (iii.) die beiden Komponenten so in Kontakt gebracht, dass die jeweils komplementären Bindungsfunktionen im Bereich der Molekülenden der ersten und zweiten Komponente so verknüpfen, insbesondere konjugieren, dass als Vernetzer ein lineares Konjugat aus mindestens einer ersten Komponente und mindestens einer damit linear verknüpften zweiten Komponente gebildet wird, wobei im Bereich der beiden Molekülenden des Konjugats, die aus erster und/oder zweiter Komponente gebildet werden, jeweils zumindest eine Bindungsfunktion des ersten Typs (A) zur Vernetzungsreaktion zur Bildung des Hydrogels vorhanden sind. Es versteht sich dass zur Unterdrückung der Selbstkonjugation mindestens eine Bindungsfunktion in derjenigen Komponente des Konjugats, die an ihren Molekülenden jeweils komplementäre Bindungsfunktionen aufweist, die miteinander selbst konjugieren können, zunächst durch eine Schutzgruppe geschützt vorliegt.

In einer ersten besonderen Ausgestaltung sieht die Erfindung ein Verfahren vor, das zumindest die folgenden Schritte enthält oder bevorzugt daraus besteht: In einem ersten Schritt wird eine erste Komponente des Vernetzers bereitgestellt, die im Bereich beider Molekülenden der Komponente jeweils zumindest eine Bindungsfunktion des ersten Typs (A) aufweist. Diese erste Komponente ist, in einer ersten Variante, das hochmolekulare lineare Polymer oder, in einer zweiten Variante, das Peptid.

Weiter wird die jeweils andere Komponente des erfindungsgemäßen Vernetzers bereitgestellt. In der ersten Variante ist dies das Peptid, in der zweiten Variante ist dies das hochmolekulare lineare Polymer. Die zweite Komponente weist im Bereich von deren ersten Molekülende ebenfalls zumindest eine Bindungsfunktion des ersten Typs (A), im Bereich von deren anderen Molekülende aber mindestens eine dazu komplementäre Bindungsfunktion des zweiten Typs (B) auf. Die beiden Komponenten werden in Kontakt gebracht, und zwar unter Bedingungen, die eine lineare Konjugation zumindest einer der Bindungsfunktionen des ersten Typs (A) der ersten Komponente mit zumindest einer der Bindungsfunktionen des komplementären zweiten Typs (B) der zweiten Komponente ermöglichen. Dabei wird als Vernetzer ein lineares Konjugat der beiden Komponenten oder ein Konjugat-Gemisch erhalten, welches jeweils mindestens eine im Bereich der beiden Molekülenden des hergestellten Konjugats lokalisierten Bindungsfunktion des ersten Typs (A) aufweist.

Gegenstand der Erfindung ist demgemäß ein Verfahren enthaltend die Schritte:
- Bereitstellen einer ersten Komponente mit jeweils im Bereich von deren beiden Molekülenden lokalisierten Bindungsfunktionen des ersten Typs (A), wobei die erste Komponente entweder (a) ein hierin charakterisiertes Polymermolekül oder (b) ein hierin charakterisiertes Peptidmolekül ist;
- Bereitstellen einer zweiten Komponente mit einer im Bereich von deren einen Molekülende lokalisierten Bindungsfunktion des ersten Typs (A) und mit einer im Bereich von deren anderen Molekülende lokalisierten Bindungsfunktion des zweiten Typs (B), wobei die zweite Komponente das jeweils andere Molekül gemäß (a) oder (b) ist; und
- Inkontaktbringen der ersten Komponente mit der zweiten Komponente unter Bedingungen, die die lineare Konjugation einer Bindungsfunktion des ersten Typs (A) der ersten Komponente mit der Bindungsfunktion des zweiten Typs (B) der zweiten Komponente ermöglichen, so dass als Polymervernetzer ein lineares Konjugat oder Konjugat-Gemisch mit jeweils im Bereich der beiden Molekülenden des Konjugats lokalisierter Bindungsfunktion des ersten Typs (A) gebildet wird.

Es wird also Typ (A) und (A) bindungsfunktionalisierte erste Komponente mit Typ (B) und (A) bindungsfunktionalisierter zweiter Komponente in Kontakt gebracht wird, um den Typ (A) und (A) bindungsfunktionalisierten Polymervernetzer zu bilden.

Die Erfindung sieht dazu bevorzugt vor, dass die mindestens eine Bindungsfunktion in derjenigen Komponente des Konjugats, die an ihren Molekülenden jeweils komplementäre Bindungsfunktionen (A) und (B), die miteinander selbst konjugieren können, trägt, zunächst geschützt vorliegt, um eine komponenteninterne Bindung (Selbstkonjugation) zu verhindern. Bevorzugt wird in dem Schritt der Bereitstellung der ersten Komponente die eine Bindungsfunktion mit einer Schutzgruppe zur Unterdrückung der Selbstkonjugation versehen oder weist diese auf. Es wird dann, bevorzugt im Anschluss an das Inkontaktbringen dieser Komponente mit der anderen Komponente, also nachdem sich das Konjugat gebildet hat, die Schutzgruppe von der einen Bindungsfunktion der ersten Komponente entfernt. In einer ersten Ausgestaltung der Erfindung ist die erste Komponente des Vernetzers das Peptidmolekül. Dieses trägt bevorzugt an seinem einen Molekülende die Bindungsfunktion des ersten Typs (A) und an seinem anderen Molekülende die dazu komplementäre Bindungsfunktion des zweiten Typs (B). In einer Variante ist die Bindungsfunktion des ersten Typs (A) durch eine Schutzgruppe geschützt. In einer Variante davon ist die Bindungsfunktion des ersten Typs (A) eine Thiolfunktion. In dieser Variante ist die Thiolfunktion durch eine Schutzgruppe in an sich bekannter Weise geschützt. Bevorzugt ist die Schutzgruppe eine Tert-Butylgruppe.

In dieser Ausführung der Erfindung wird ein erfindungsgemäßes Konjugat erhalten, welches am jeweiligen Molekülende eine Bindungsfunktion trägt, die ausgewählt ist aus einer auch in der intramolekularen Konjugationsreaktion eingesetzten nukleophilen Gruppe und der elektrophilen Doppelbindung. Wird für die intramolekulare Konjugation zwischen Polymermolekül und Peptidmolekül das komplementäre Bindungsfunktionspaar Maleimid- und Thiolfunktion eingesetzt, so weist das erfindungsgemäße Konjugat an seinen Molekülenden endständig entweder jeweils eine Thiolfunktion oder jeweils eine Maleimidfunktion auf. Mit diesem erfindungsgemäßen Vernetzer können Polymere vernetzt werden, mit der entsprechenden komplementären Bindungsfunktion funktionalisiert sind.

Die Erfindung betrifft auch Vernetzer, die nach den hierin beschriebenen erfindungsgemäßen Verfahren herstellbar sind, beziehungsweise durch diese unmittelbar hergestellt werden. Diese sind hierin näher charakterisiert.

Die Erfindung umfasst auch einen Polymervernetzer, der ein Konjugat-Gemisch ist aus Konjugaten, die in der Konjugationsreaktion der hochmolekularen Polymerkomponente mit der Peptidkomponente unterschiedlichen Molverhältnissen der Ausgangskomponenten gebildet werden. Ein erstes erhältliches Konjugat als Bestandteil des Konjugat-Gemisches besteht aus Polymerkomponente und Peptidkomponente in einem Molverhältnis von 1:1. Ein anderes Konjugat besteht aus Polymerkomponente und Peptidkomponente in einem Molverhältnis von 1:2. Ein anderes Konjugat besteht aus Polymerkomponente und Peptidkomponente in einem Molverhältnis von 2:1.

In einer Variante dieser Ausführung enthält das erhaltene Vernetzer-Gemisch zusätzlich auch die nicht konjugierte bindungsfunktionalisierte hochmolekulare Polymerkomponente. Der Anteil an nicht konjugierter Komponente im Produktgemisch ist abhängig von der Stöchiometrie der eingesetzten Edukte. In einer bevorzugten Variante werden die Edukte in einem äquimolaren Verhältnis eingesetzt. Das Produktgemisch enthält demgemäß zu jeweils etwa einem Drittel Konjugate mit äquimolarem Verhältnis von Peptid- und Polymerkomponente, zu einem weiteren Drittel Konjugate, worin jeweils zwei Peptidkomponenten mit einer Polymerkomponente konjugiert sind, und als letztes Drittel die unkonjugierte Polymerkomponente. Alle drei Komponenten des Produktgemisches haben gemeinsam, dass sie am jeweiligen Molekülende dieselbe Bindungsfunktionalisierung aufweisen und jeweils als Vernetzer dienen können. Es versteht sich, dass in den hier angeführten beispielhaften Ausgestaltungen von einem gemäß der ersten Variante des Herstellverfahrens ausgestalteten Vernetzermolekül ausgegangen wird; Polymerkomponente und Peptidkomponente sind aber entsprechend der zweiten Variante des Verfahrens austauschbar, ohne dass dadurch die Lehre der vorgestellten Erfindung verlassen wird.

Die in einer Variante der Erfindung vorgesehene Biospaltbarkeit ist nur bei denjenigen Vernetzern, die eine spaltbare Komponente, aufweisen, gewährleistet. Die Spaltbarkeit des damit hergestellten Hydrogels ist von dem Anteil an spaltbaren Vernetzermolekülen, und insbesondere von dem Verhältnis von spaltbarem Vernetzer zu nicht spaltbarem Vernetzer abhängig. Es versteht sich daher, dass der Anteil an nicht konjugierter Polymerkomponente in dem hergestellten Vernetzer-Gemisch einen gewissen Anteil nicht überschreiten darf, um die Biospaltarbeit des damit vernetzten Hydrogels nicht nachteilig zu beeinflussen. Bevorzugt ist vorgesehen, dass der Anteil an unkonjugierter Polymerkomponente im Vernetzer-Gemisch, 50 % oder weniger, bevorzugt 33 % oder weniger, beträgt.

In einer alternativen zweiten besonderen Ausgestaltung sind die Komponenten im erfindungsgemäßen Vernetzermolekül intramolekular über eine andere Verknüpfungsreaktion verbunden, als für die Vernetzung mit einem bindungsfunktionalisierten Polymer bereitgestellt wird. In dieser Ausführung wird eine erste Komponente des Vernetzers bereitgestellt, die mindestens eine im Bereich von deren einen Molekülende lokalisierten Bindungsfunktion des ersten Typs (A) und mindestens eine im Bereich von deren anderen Molekülende lokalisierten Bindungsfunktion eines dritten Typs (C) aufweist, der mit dem ersten Typ (A) keine Verknüpfung eingeht. So kann die Selbstkonjugation innerhalb dieser Komponente verhindert werden. In einer ersten Variante ist diese erste Komponente des Vernetzers das hochmolekulare lineare Polymer; in einer alternativen zweiten Variante ist diese erste Komponente das Peptid.

Es wird eine zweite Komponente bereitgestellt, welche jeweils mindestens eine im Bereich von deren beiden Molekülenden lokalisierten Bindungsfunktion eines vierten Typs (D) aufweist, wobei die Bindungsfunktion dieses vierten Typs (D) zu der Bindungsfunktion des dritten Typs (C) komplementär ist, mit dem ersten Typ (A) aber keine Verknüpfung eingehen kann. In der ersten Variante ist die zweite Komponente das Peptid; in der zweiten Variante ist die zweite Komponente das hochmolekulare lineare Polymer.

Die erste Komponente wird mit der zweiten Komponente in Kontakt gebracht, und zwar unter Bedingungen, die die lineare Konjugation der Bindungsfunktion des dritten Typs (C) der ersten Komponente mit der Bindungsfunktion des vierten Typs (D) der zweiten Komponente ermöglichen. Dabei werden ein lineares Konjugat und gegebenenfalls ein Konjugat-Gemisch mit jeweils einer im Bereich der beiden Molekülenden des Konjugats lokalisierten Bindungsfunktion vom ersten Typ (A) gebildet. Die Verbindung zwischen drittem Typ (C) und viertem Typ (D) dient ausschließlich der intramolekularen Verknüpfung; an der intramolekularen Verknüpfung der Komponenten des Vernetzermoleküls sind Bindungsfunktionen vom ersten Typ(A) nicht beteiligt.

Gegenstand der Erfindung ist demgemäß ein Verfahren enthaltend die Schritte:
- Bereitstellen einer ersten Komponente, die eine im Bereich von deren einen Molekülende lokalisierte Bindungsfunktion des ersten Typs (A) und eine im Bereich von deren anderen Molekülende lokalisierte Bindungsfunktion eines dritten Typs (C) aufweist, wobei die erste Komponente entweder (a) ein hierin charakterisiertes Polymermolekül oder (b) ein hierin charakterisiertes Peptidmolekül ist;
- Bereitstellen einer zweiten Komponente mit jeweils im Bereich von deren beiden Molekülenden lokalisierten Bindungsfunktionen eines vierten Typs (D), die zu der Bindungsfunktion des dritten Typs (C) komplementär sind, wobei die zweite Komponente das jeweils andere Molekül gemäß (a) oder (b) ist; und
- Inkontaktbringen der ersten Komponente mit der zweiten Komponente unter Bedingungen, die die lineare Konjugation der Bindungsfunktion des dritten Typs (C) der ersten Komponente mit der Bindungsfunktion des vierten Typs (D) der zweiten Komponente ermöglichen, so dass als Polymervernetzer ein lineares Konjugat mit jeweils im Bereich der Molekülenden des Konjugats lokalisierter Bindungsfunktion vom Typ (A) gebildet wird.

Es wird also Typ (A) und (C) bindungsfunktionalisierte erste Komponente mit Typ (D) und (D) bindungsfunktionalisierter zweiter Komponente in Kontakt gebracht wird, um den Typ (A) und (A) bindungsfunktionalisierten Polymervernetzer zu bilden.

In einer bevorzugten Variante davon trägt die Polymerkomponente im Bereich des einen Molekülendes eine Bindungsfunktion eines ersten Typs (A), beispielsweise und bevorzugt eine Thiol-Funktion und im Bereich des anderen Molekülendes eine Bindungsfunktion eines dritten Typs (C), beispielsweise und bevorzugt eine Azid-Funktion. Die Peptidkomponente weist jeweils eine mindestens im Bereich von beiden Molekülenden lokalisierte Bindungsfunktion eines vierten Typs (D), die zu der Bindungsfunktion des dritten Typs (C) des Polymers komplementär ist; beispielsweise ist diese eine Alkyn-Funktion.

In einer dritten besonderen Ausgestaltung wird ebenfalls eine erste Komponente des Vernetzers bereitgestellt, die mindestens eine im Bereich von deren einen Molekülende lokalisierten Bindungsfunktion des ersten Typs (A) und mindestens eine im Bereich von deren anderen Molekülende lokalisierten Bindungsfunktion des dritten Typs (C) aufweist. In einer ersten Variante ist diese erste Komponente des Vernetzers das hochmolekulare lineare Polymer; in einer alternativen zweiten Variante ist diese erste Komponente das Peptid.

Weiter wird dieser weiteren Ausführung aber eine zweite Komponente bereitgestellt, welche mindestens eine im Bereich von deren einen Molekülende lokalisierten Bindungsfunktion des ersten Typs (A) und mindestens eine im Bereich von deren anderen Molekülende lokalisierten Bindungsfunktion des vierten Typs (D) aufweist. In der ersten Variante ist die zweite Komponente das Peptid; in der zweiten Variante ist die zweite Komponente das hochmolekulare lineare Polymer.

Die erste Komponente wird mit der zweiten Komponente in Kontakt gebracht, und zwar unter Bedingungen, die die lineare Konjugation der Bindungsfunktion des dritten Typs (C) der ersten Komponente mit der Bindungsfunktion des vierten Typs (D) der zweiten Komponente ermöglichen. Dabei wird ein lineares Konjugat mit jeweils einer im Bereich der beiden Molekülenden des Konjugats lokalisierten Bindungsfunktion vom ersten Typ (A) gebildet. Die Verbindung zwischen drittem Typ (C) und viertem Typ (D) dient ausschließlich der intramolekularen Verknüpfung; an der intramolekularen Verknüpfung der Komponenten des Vernetzermoleküls sind Bindungsfunktionen vom ersten Typ(A) nicht beteiligt.

Gegenstand der Erfindung ist demgemäß ein Verfahren enthaltend die Schritte:
- Bereitstellen einer ersten Komponente, die eine im Bereich von deren einen Molekülende lokalisierte Bindungsfunktion des ersten Typs (A) und eine im Bereich von deren anderen Molekülende lokalisierte Bindungsfunktion eines dritten Typs (C) aufweist, wobei die erste Komponente entweder (a) ein hierin charakterisiertes Polymermolekül oder (b) ein hierin charakterisiertes Peptidmolekül ist;
- Bereitstellen einer zweiten Komponente mit jeweils im Bereich von deren beiden Molekülenden lokalisierten Bindungsfunktionen eines vierten Typs (D), die zu der Bindungsfunktion des dritten Typs (C) komplementär sind, wobei die zweite Komponente das jeweils andere Molekül gemäß (a) oder (b) ist; und
- Inkontaktbringen der ersten Komponente mit der zweiten Komponente unter Bedingungen, die die lineare Konjugation der Bindungsfunktion des dritten Typs (C) der ersten Komponente mit der Bindungsfunktion des vierten Typs (D) der zweiten Komponente ermöglichen, so dass als Polymervernetzer ein lineares Konjugat mit jeweils im Bereich der Molekülenden des Konjugats lokalisierter Bindungsfunktion vom Typ (A) gebildet wird.

Es wird also Typ (A) und (D) bindungsfunktionalisierte erste Komponente mit Typ (C) und (D) bindungsfunktionalisierter zweiter Komponente in Kontakt gebracht wird, um den Typ (A) und (A) bindungsfunktionalisierten Polymervernetzer zu bilden.

In einer bevorzugten Variante davon trägt die Polymerkomponente im Bereich des einen Molekülendes eine Bindungsfunktion des ersten Typs (A), beispielsweise und bevorzugt eine Thiol-Funktion, und im Bereich des anderen Molekülendes eine Bindungsfunktion des dritten Typs (C), beispielsweise und bevorzugt eine Azid-Funktion. Die Peptidkomponente trägt im Bereich des einen Molekülendes eine Bindungsfunktion des ersten Typs (A), beispielsweise und bevorzugt eine Thiol-Funktion, und im Bereich des anderen Molekülendes eine Bindungsfunktion des dritten Typs (D), beispielsweise und bevorzugt eine Alkyn-Funktion.

Gemäß dieser alternativen Ausgestaltungen stellt die Erfindung bevorzugt einen Vernetzer bereit, worin Peptidkomponente und Polymerkomponente intramolekular über eine Bindungsfunktion eines Typs (C) und eines Typs (D), beispielsweise und bevorzugt über die Verknüpfung einer Azid-Funktion mit einer Alkyn-Funktion, verbunden sind. Am jeweiligen Molekülende des Vernetzers sind dabei Bindungsfunktionen des Typs (A) lokalisiert, die bei der späteren Verwendung zur Vernetzung bindungsfunktionalisierter Polymere mit komplementären Bindungsfunktionen vom Typ (B) verknüpft werden können.

Es versteht sich, dass neben den vorgenannten konkreten Ausgestaltungen weitere Ausgestaltungen denkbar sind, die der Fachmann ohne weiteres daraus ableiten kann. Besonders können auch jeweils Mischungen unterschiedlich bindungsfunktionalisierter erster oder zweiter Komponenten eingesetzt werden. Beispielhaft dafür ist ein Gemisch aus Typ (A) und (D) bindungsfunktionalisierter erster Komponente und Typ (A) und (C) bindungsfunktionalisierter erster Komponente, die mit Typ (C) und (D) bindungsfunktionalisierter zweiter Komponente in Kontakt gebracht wird, um den Typ (A) und (A) bindungsfunktionalisierten Polymervernetzer zu bilden.

Besonders versteht sich, dass die erste Komponente des Polmervernetzers in einer ersten Variante die Peptidkomponente als auch in einer zweiten Variante die Polymerkomponente sein kann, wobei die zweite Komponente des Polmervernetzers in dieser ersten Variante die Polymerkomponente und in der zweiten Variante die Peptidkomponente ist, auch wenn hierin die Erfindung beispielhaft nur anhand einer der beiden Varianten illustriert ist.

Weiter betrifft die Erfindung Verfahren zur Herstellung eines Hydrogels. Dieses Verfahren enthält zumindest die folgenden Schritte oder besteht daraus: Zunächst wird ein erfindungsgemäßer oder erfindungsgemäß hergestellter oder erfindungsgemäß herstellbarer Polymervernetzer bereitgestellt. Alternativ werden die Schritte der vorstehend charakterisierten Verfahren zur Herstellung des erfindungsgemäßen Polymervernetzers durchgeführt. Daneben wird ein komplementär bindungsfunktionalisiertes Polymer bereitgestellt. In dem Verfahren werden der Polymervernetzer mit dem Polymer in Kontakt gebracht, und zwar unter Bedingungen, die eine Konjugation der Bindungsfunktionen des Polymervernetzers mit den dazu komplementären Bindungsfunktionen des Polymers ermöglichen, sodass ein Hydrogel aus vernetztem Polymer gebildet wird.

Ein zur Herstellung des Hydrogels verwendetes Polymer ist bevorzugt ausgewählt aus der Gruppe der Polymere bestehend aus: Polyethylenglykol (PEG), Polypropylenglykol (PPG), Polyvinylalkohol (PVA), Polyvinylpyrrolidon (PVP), Dextran, Pullulan, Methylcellulose, Amylose, Amylopektin, Glykogen und Albuminen, besonders Serumalbuminen sowie Mischungen zweiter oder mehrerer davon. Ein besonders bevorzugtes Polymer ist PEG. Ein alternativ besonders bevorzugtes Polymer ist PVA. Ein anderes bevorzugtes Polymer ist Dextran. Besonders bevorzugt sind Serumalbumin oder Serumprotein, die sich zu besonders biokompatiblen und biomimetischen hydrophilen Gelen vernetzen lassen. Das Serumalbumin beziehungsweise das Serumprotein werden in an sich bekannter Weise bevorzugt aus Säugetieren, besonders bevorzugt aus dem menschlichen Organismus (humanes Blutserum) oder aus Rinderserum gewonnen. Ein bevorzugtes funktionalisiertes Polymer, das im Zusammenhang mit dem erfindungsgemäßen linearen Konjugat als Vernetzermolekül eingesetzt werden kann, ist Maleimid-modifiziertes Rinderserumalbumin. Der Fachmann kennt weitere, besonders hydrophile Polymere, die in bindungsfunktionalisierter Form mit dem Vernetzer zur Bildung eines Hydrogels verwendet werden können und die biokompatible sind, falls die Anwendung des Hydrogels im Zusammenhang mit biologischen Zellen oder Geweben gewünscht ist.

Es versteht sich, dass die Erfindung nicht auf diese Anwendung bei biokompatiblen Hydrogelen beschränkt ist. Weitere Anwendungen liegen im Bereich der physikalischen und chemischen Analyse sowie in der präparativen Chemie.

Gegenstand der Erfindung ist auch die Verwendung des erfindungsgemäßen Polymervernetzers zur Reduktion der Vernetzerkonzentration im hergestellten Hydrogel. Ein besonderer Aspekt der Erfindung ist die Verwendung des Vernetzers zur Erhöhung des Wasseranteils im Hydrogel.

Gegenstand der Erfindung ist auch ein Hydrogel, das mit den vorgenannten Verfahren herstellbar ist beziehungsweise unmittelbar damit hergestellt wird. Gegenstand der Erfindung ist auch ein Hydrogel, das den erfindungsgemäßen Polymervernetzer, darin gebunden, enthält, und zwar bevorzugt in einem Anteil von 10 mmol/L oder weniger, bevorzugt von 3 mmol/L bis 10 mmol/L, bezogen auf das Hydrogel. In einer ersten Variante enthält das Hydrogel keine weiteren Vernetzerkomponenten. Die gelbildenden Komponenten des Hydrogels sind in einer bevorzugten Ausführung beschränkt auf funktionalisiertes Polymer und erfindungsgemäße Vernetzer dieses Polymers.

In einer anderen Variante enthält das Hydrogel neben dem erfindungsgemäßen Vernetzer weitere Vernetzerkomponenten. In dieser Variante sind die Hydrogele bevorzugt durch Vernetzung mit Vernetzerzusammensetzungen, die den erfindungsgemäßen Vernetzer neben mindestens einer weiteren Vernetzerkomponente enthalten, hergestellt. Eine Vernetzerzusammensetzung kann neben dem erfindungsgemäßen Vernetzermolekül einen herkömmlichen Polymervernetzter wie Dithio-PEG enthalten.

Die Hydrogele sind besonders geeignet für die Kultivierung, insbesondere autologer, allogener oder xenogener Zellen, besonders Primärzellen, insbesondere mesenchymaler Stammzellen, die darin durch geeignete Mittel beispielsweise zu Knorpelzellen, besonders zu Chondrozyten, differenzieren können. Die Erfindung ist aber nicht auf die Verwendung zur Kultivierung solcher Zellen beschränkt. Dazu zählen allgemein primäre Zellen, wie somatische Stammzellen oder zu pluripotenten Zellen rückprogrammierte somatische Zellen "induced pluripotent stem cells" (iPS) sowie Zelllinien. Ein besonderes Einsatzgebiet ist auch die Verwendung bei Tumorzellen. Diese können in dem bevorzugt spaltbaren Hydrogel wandern.

Dazu zählen beispielsweise auch Fibroblastenzellen, die, gegebenenfalls unter Verwendung weiterer Biomodulatoren und Wachstumsfaktoren, die fibroblastentypische Morphologie entwickeln. Die Erfinder fanden überraschend, dass die Kultivierung von Fibroblastenzellen in erfindungsgemäß herstellbaren Hydrogelen die Differenzierung in eine fibroblastentypische Morphologie unterstützt beziehungsweise in Differenzierungsstatus über die Dauer der Kultivierung erhält.

Das erfindungsgemäße oder erfindungsgemäß herstellbare Hydrogel liegt bevorzugt in Form einer, bevorzugt biospaltbaren, Zellkulturmatrix oder eines Kulturgels vor.

Gegenstand der Erfindung ist auch die Verwendung des erfindungsgemäßen Polymervernetzers als Bestandteil des Hydrogels zur Kultivierung von Zellen.

Gegenstand der Erfindung ist auch die Verwendung des erfindungsgemäßen Polymervernetzers als Bestandteil eines Kits zur Herstellung eines Hydrogels insbesondere zur Kultivierung von Zellen, wobei das Kit neben dem Polymervernetzer zumindest auch das zu vernetzende Polymer enthält.

Nachfolgend wird die Erfindung anhand von Beispielen und zugehörigen Figuren näher beschrieben, ohne dass diese beschränkend zu verstehen wären:

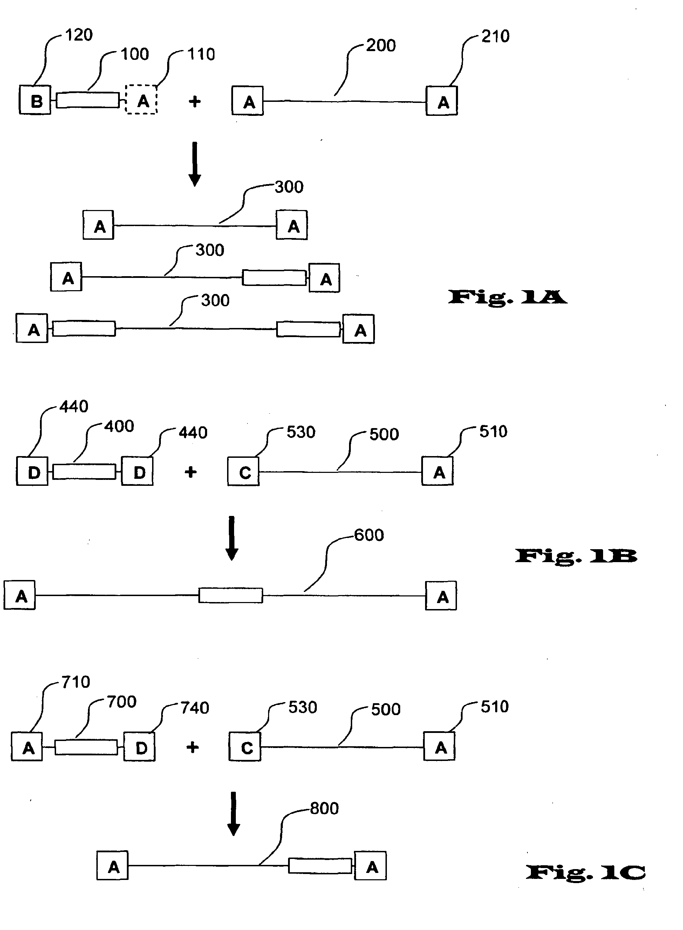

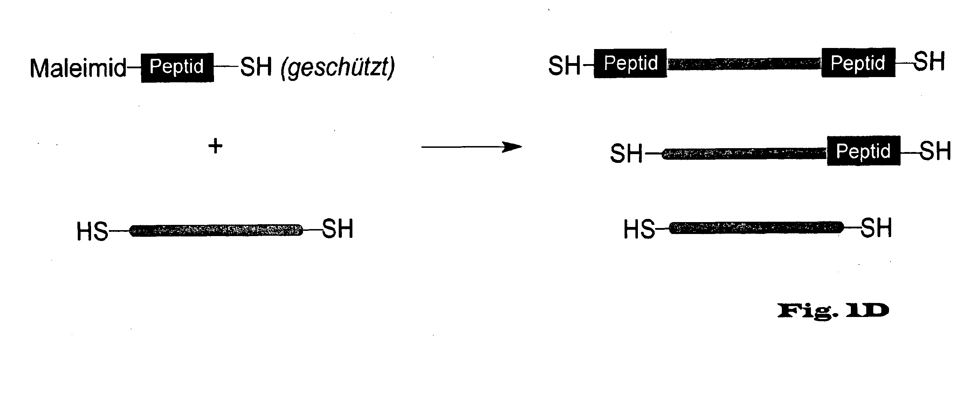

Figur 1ABC zeigt schematische Darstellungen der Strategie zur Herstellung der erfindungsgemäßen linearen Konjugate. Es ist schematisch die Reaktion äquimolarer Mengen von Peptidkomponente und PolymerKomponente illustriert:
   Gemäß Figur 1A wird ein hochmolekulares lineares Polymer (200) als die Polymerkomponente des Vernetzers (300) bereitgestellt. Das hochmolekulare lineare Polymer weist im Bereich beider Molekülenden zumindest eine Bindungsfunktion des ersten Typs (A) (210) auf. Weiter wird ein Peptid (100) als Peptidkomponente bereitgestellt. Dieses weist im Bereich von dessen ersten Molekülende eine Bindungsfunktion des ersten Typs (A) (110) und im Bereich von dessen anderen Molekülende eine Bindungsfunktion des komplementären zweiten Typs (B) (120) auf. Die Bindungsfunktion vom ersten Typ (A) (110) ist zunächst durch eine Schutzgruppe gegen Selbstkonjugation mit der komplementären Bindungsfunktion vom Typ (B) (120) geschützt. Die Polymerkomponente (200) und die Peptidkomponente (100) werden in Kontakt gebracht, und zwar unter Bedingungen, die die lineare Konjugation einer der Bindungsfunktionen des ersten Typs (A) (210) des Polymeres (200) mit der Bindungsfunktion des komplementären zweiten Typs (B) (120) des Peptids (100) ermöglichen, sodass als Vernetzer ein lineares Konjugat (300) oder ein Konjugat-Gemisch (300) mit jeweils einer im Bereich der beiden Molekülenden des hergestellten Konjugats (300) lokalisierten Bindungsfunktion des Typs (A) gebildet wird.
   Gemäß Figur 1B wird ein hochmolekulares lineares Polymer (500) als Polymerkomponente des Vernetzers bereitgestellt, das mit einer im Bereich des einen Molekülendes lokalisierten Bindungsfunktion des ersten Typs (A) (510) und mit einer im Bereich des anderen Molekülendes lokalisierten Bindungsfunktion eines dritten Typs (C) (530) ausgestattet ist. Dabei ist die Bindungsfunktion des dritten Typs (C) (530) mit der Bindungsfunktion des ersten Typs (A) (510) nicht komplementär und kann mit dieser keine Verknüpfung eingehen oder ein Konjugat bilden. Weiter wird ein lineares Peptid (400) als Peptidkomponente bereitgestellt, welches jeweils eine im Bereich von dessen beiden Molekülenden lokalisierten Bindungsfunktion eines vierten Typs (D) (440) ausgestattet ist, wobei die Bindungsfunktion des vierten Typs (D) (440) zu der Bindungsfunktion des dritten Typs (C) (530) komplementär ist. Die Polymerkomponente (500) wird mit der Peptidkomponente (400) in Kontakt gebracht, und zwar unter Bedingungen, die die lineare Konjugation der Bindungsfunktion des dritten Typs (C) (530) des Polymers (500) mit der Bindungsfunktion des vierten Typs (D) (440) des Peptids (400) ermöglichen. Es wird als Vernetzer ein lineares Konjugat (600) mit jeweils einer im Bereich der beiden Molekülenden des Konjugats (600) lokalisierten Bindungsfunktion vom Typ (A) gebildet.
   Gemäß Figur 1C wird das hochmolekulare lineare Polymer (500) als gemäß Figur 1B bereitgestellt. Weiter wird ein lineares Peptid (700) als Peptidkomponente bereitgestellt, welches mit einer im Bereich des einen Molekülendes lokalisierten Bindungsfunktion des ersten Typs (A) (710) und mit einer im Bereich des anderen Molekülendes lokalisierten Bindungsfunktion des vierten Typs (D) (540) ausgestattet ist. Die Polymerkomponente (500) wird mit der Peptidkomponente (700) in Kontakt gebracht, und zwar unter Bedingungen, die die lineare Konjugation der Bindungsfunktion des dritten Typs (C) (530) des Polymers (500) mit der Bindungsfunktion des vierten Typs (D) (740) des Peptids (700) ermöglichen. Es wird als Vernetzer ein lineares Konjugat (800) mit jeweils einer im Bereich der beiden Molekülenden des Konjugats (800) lokalisierten Bindungsfunktion vom Typ (A) gebildet.
Figur 1 D zeigt eine konkrete Ausgestaltung des Verfahrens nach Figur 1A.
   Die Peptidkomponente des Vernetzers wird bevorzugt mittels der Festphasenpeptidsynthese-Technik synthetisiert. Eine Variante ist dabei die DIC/CI-HOBt-Kupplungsmethode unter Verwendung der Reagenzien Diisopropylcarbodiimid (DIC) und Hydroxybenzotriazol (HOBT). Die Peptidsynthese findet in an sich bekannter Weise statt. Bevorzugt werden als Synthesebausteine 9-Fluorenylmetoxycarbonyl (Fmoc)-konjugierte Aminosäuren oder Aminosäurederivate eingesetzt. Als Synthesereagenzien dienen Fmoc-3,6-Dioxaoctansäure und Fmoc-(2,4-Dinitrophenyl)-Diamino-Propionsäure. Die Festphasenpeptidsynthese findet bevorzugt an einem Harzträger, beispielsweise Sieberharz, statt. bevorzugt wird am C-terminalen Ende eine chemisch geschützte Thiolfunktion vorgesehen. Zur Einführung einer N-terminalen Maleimidfunktion wird bevorzugt N-Maleoyl-β-Aminosäure, beispielsweise N-Maleoyl-β-Alanin, eingesetzt. Die Maleimidfunktionalisierung erfolgt bevorzugt an dem am Peptidyl-Harz immobilisierten Peptids.
   In einer Variante wird das Peptidyl-Harz anschließend in an sich bekannter Weise in einer Lösemittelreihe (Dichlormethan, Dimethylformamid, Dichlormethan und Diethylether) gewaschen und anschließend, bevorzugt im Hochvakuum, getrocknet. Die Abspaltung des Peptids vom Harz erfolgt in an sich bekannter Weise, bevorzugt mittels Lösemittelreihe enthaltend Wasser, Triisopropylsilan und Triflouressigsäure. Das Peptid wird bevorzugt mit Diethylether ausgefällt und anschließend im Lösungsmittel gewaschen. Bevorzugt ist zusätzlich vorgesehen, dass das Peptid die Lösungsmittel, beispielsweise Tert-Butanol und Wasser, aufgenommen und, bevorzugt anschließend, lyophylisiert wird. Auf diese Weise wird ein synthetisches Peptid erhalten, dessen C-terminales Ende mit einer chemisch geschützten Thiolfunktion und am N-terminalen Ende mit einer Maleimidfunktion versehen ist.
   Zur Herstellung eines erfindungsgemäßen linearen Konjugats wird das synthetisierte und funktionalisierte lineare Peptid mit der PEG-Komponente in Verbindung gebracht. Die PEG-Komponente ist ein lineares PEG, welches am jeweiligen Molekülende jeweils eine Thiolfunktion aufweist. Die Peptidkomponente und die PEG-Komponente werden erfindungsgemäß unter Bedingungen in Verbindung gebracht, welche die Konjugation und damit die Bildung von linearen Konjugaten von Peptidkomponente und PEG-Komponente ermöglichen. Bevorzugt werden dabei drei Reaktionsprodukte erhalten: ein erstes Konjugationsprodukt enthält eine PEG-Komponente und zwei jeweils am Ende der PEG-Komponente angefügte Peptidkomponenten, worin jeweils die Maleimidfunktion der Peptidkomponente mit einer endständigen Thiolfunktion des linearen PEG konjugiert ist. Das molare Verhältnis von Peptidkomponente und PEG-Komponente in diesem linearen Konjugat beträgt 2:1. Ein weiteres Reaktionsprodukt ist ein lineares Konjugat, worin eine PEG-Komponente an einem Ende mit einer Peptidkomponente konjugiert ist, und zwar über die Maleimidgruppe des Peptids mit der Thiolgruppe am einen Ende des PEG-Moleküls. Das Molverhältnis von Peptidkomponente zu PEG-Komponente in diesem Konjugat beträgt 1:1.
   In einer bevorzugten Variante werden die Reaktionspartner in etwa äquimolaren Mengen zur Konjugation gebracht. Die Erfindung sieht dabei vor, dass aufgrund der molaren Verhältnisse der Komponenten im Konjugationsprodukt im Produktgemisch etwa 33 % doppelt konjugiertes Konjugat (Molverhältnis Peptidkomponente zu PEG-Komponente: 2:1), etwa 33 % einfach konjugiertes Konjugat (Molverhältnis Peptidkomponente zu PEG-Komponente: 1:1) und etwa 33 % unkonjugiertes Dithiol-PEG enthalten ist. Die Erfindung sieht deshalb in einer bevorzugten Ausführung auch ein Konjugat-Gemisch vor, das zumindest aus dem doppelt konjugierten Konjugat und dem einfach konjugierten Konjugat besteht und gegebenenfalls zusätzlich unkonjugiertes Dithiol-PEG enthält.
Figur 2 zeigt ein Massenspektrogramm eines zur Bildung des erfindungsgemäßen Vernetzers verwendeten synthetischen Peptids.
Figur 3 zeigt die RP-HPLC-Analyse des Peptids nach Figur 2.
Figur 4 zeigt ein Absorptionsspektrum im Bereich von 200 bis 800 nm des Peptids nach den Figuren 2 und 3.
Figur 5 zeigt die Gelfiltrationsanalyse, dargestellt als Absorption, bei einer Wellenlänge von 358 nm (Dinitrophenylsignal (Dap(Dnp))) des Peptids nach den Figuren 2, 3 und 4.
Figur 6 zeigt ein Chromatogramm bei 258 nm nach Gelfiltration des erfindungsgemäßen linearen Konjugats aus dem Peptid und einer PEG-Komponente.
Figur 7 zeigt die schematische Darstellung des enzymatischen Abbaus des erfindungsgemäßen Vernetzer durch Matrixmetalloprotease (MMP). Die Pfeilspitze zeigt auf die Schnittstelle von MMP in der Peptidkomponente. Der Chromophor (Dap(Dnp)) verbleibt im abgespaltenen Fragment der Peptidkomponente des Vernetzermoleküls.
Figur 8 zeigt Chromatogramme bei 358 nm nach Gelfiltration des erfindungsgemäßen Vernetzermoleküls vor (Control 2) und nach MMP2-Verdau (Assay 3) sowie die Enzymkontrolle (Control 1): Durch den MMP2-Verdau wird von dem erfindungsgemäßen linearen Konjugat ein kleineres Fragment abgespalten, welches bei etwa 20 ml eluiert (Assay 3).
Figur 9 zeigt Vergleichsexperimente zur Gelbildung mit unterschiedlichen Quervernetzern und Maleimid-Dextran als zu vernetzendes Polymer: Die Effizienz der Gelbildung durch das erfindungsgemäße lineare Konjugat als Vernetzer ist im Vergleich zu unkonjugiertem niedermolekularen Peptid als Vernetzer um mehr als das 3fache verbessert (Legende: K1: erfindungsgemäßes lineares Konjugat; SPS: Dithiol-Peptid; Peptid 2: niedermolekulares Kontrollpeptid mit zwei distalen Thiolfunktionen (Cystein)).
Figur 10 zeigt mikroskopische Phasenkontrastbilder von Fibroblasten (Zelllinie 3T3) in PVA-Hydrogelen nach zwei Tagen in Kultur. Zur Bildung der PVA-Hydrogele wurden erfindungsgemäßes Peptid-PEG-Konjugat (10A, C) oder PEG (10B, D) als Vernetzer in Gegenwart von 1 mmol/L Adhäsionspeptid RGD (10A, B) oder 1 mmol/L Thioglycerol (10C, D) jeweils kovalent an PVA angekoppelt.

### Beispiel 1: Synthese eines maleimid- und thiolfunktionalisierten synthetischen Peptids (erfindungsgemäß)

Es wurde Peptid Mal-GKPLGL-Dap(Dnp)-AR-Doa-Cys(StBu)-NH₂ synthetisiert als Komponente des erfindungsgemäßen Vernetzermoleküls.

Mit Hilfe der DIC/Chlor-HOBt-Kupplungsmethode wurde ein Peptid an einem Sieber-Harz (Novabiochem) mittels der Festphasenpeptidsynthese-Technik synthetisiert. Als Synthesebausteine wurden Fmoc-3,6-dioxaoktansäure und Fmoc-(2,4-dinitrophenyl)-diamino-propionsäure (Iris Biotech, Marktredwitz, Deutschland) sowie Fmoc-L-Arg(Pbf), Fmoc-L-Cys(StBu), Fmoc-Gly, Fmoc-L-Lys(Boc), Fmoc-L-Pro, Fmoc-L-Ala und Fmoc-L-Leu (Merck KGaA, Darmstadt, Deutschland) eingesetzt. Zur Einführung der N-terminalen Maleimid-Gruppe wurde N-Maleoyl-ß-Alanin, als Lösemittel wurde N,N-Dimethylformamid (Biosolve, Valkensvaard, NL) benutzt.

Das Peptidyl-Harz wurde mit Dichlormethan und DMF und anschließend mit wiederum Dichlormethan und Diethylether gewaschen und im Hochvakuum getrocknet. Anschließend wurde die Abspaltung des Peptids vom Harz mittels folgender Mischung durchgeführt: Wasser / Triisopropylsilan / Trifluoressigsäure = 5/3/92 (v / v / v)

Nach 1,5 Stunden Inkubation bei Raumtemperatur (RT) wurde das Peptid mit Diethylether ausgefällt, 1 Std. bei -20 °C aufbewahrt und das Präzipitat anschließend dreimal mit Diethylether gewaschen. Nach dem letzten Waschschritt wurde das Peptid mit Tert-Butanol / Wasser = 4 / 1 (v / v) aufgenommen und lyophilisiert.

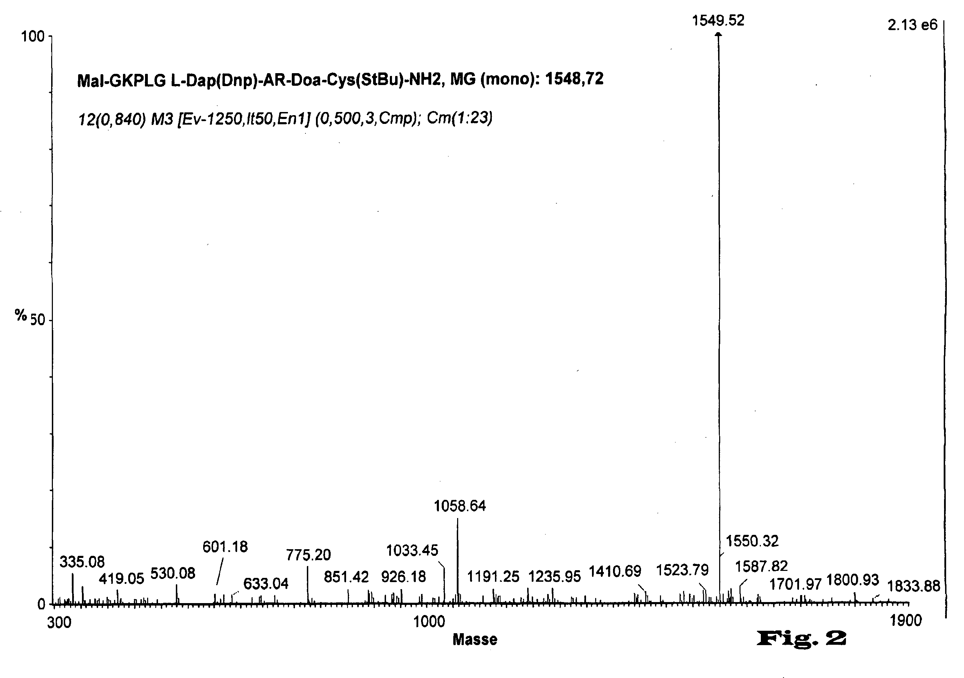

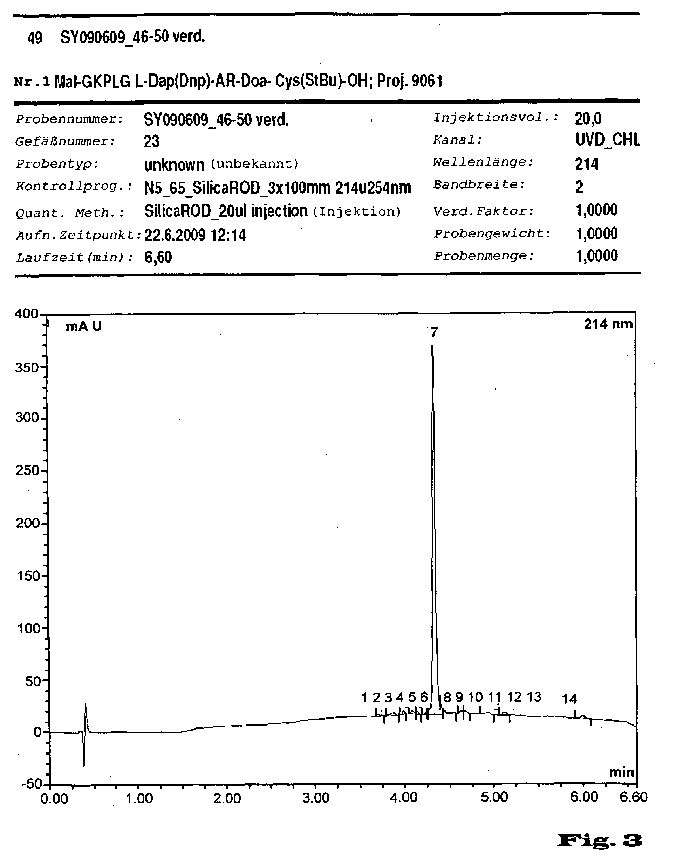

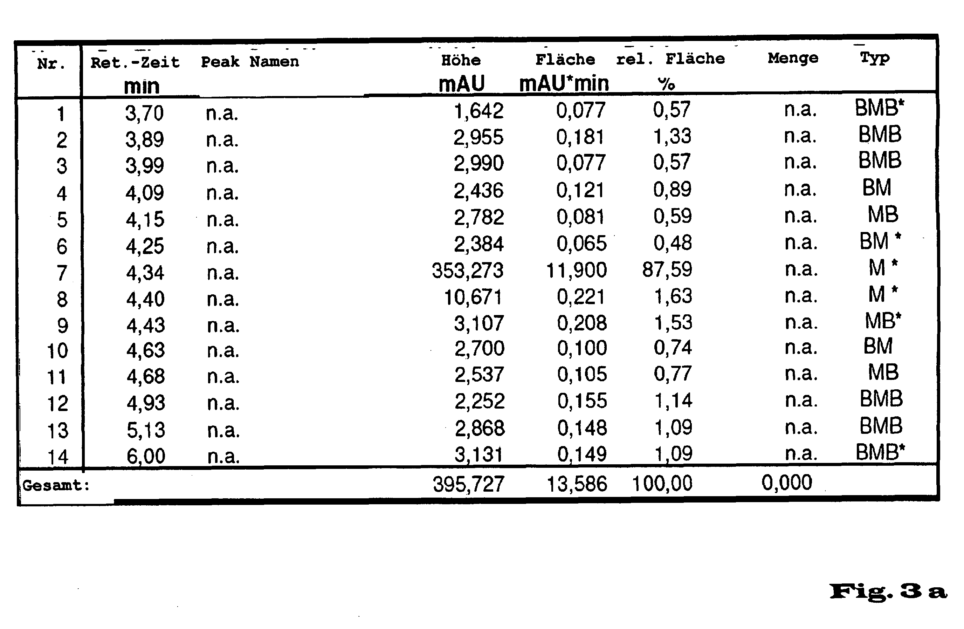

Die Charakterisierung des Produkts mit HPLC und Massenspektrometrie ergab eine Reinheit von 87,6 % (Detektion bei 214 nm); die gefundene Masse ist [M+H]⁺ = 1549,52 im Vergleich zur monoisotopischen Soll-masse von 1548,72 (Figuren 2 und 3).

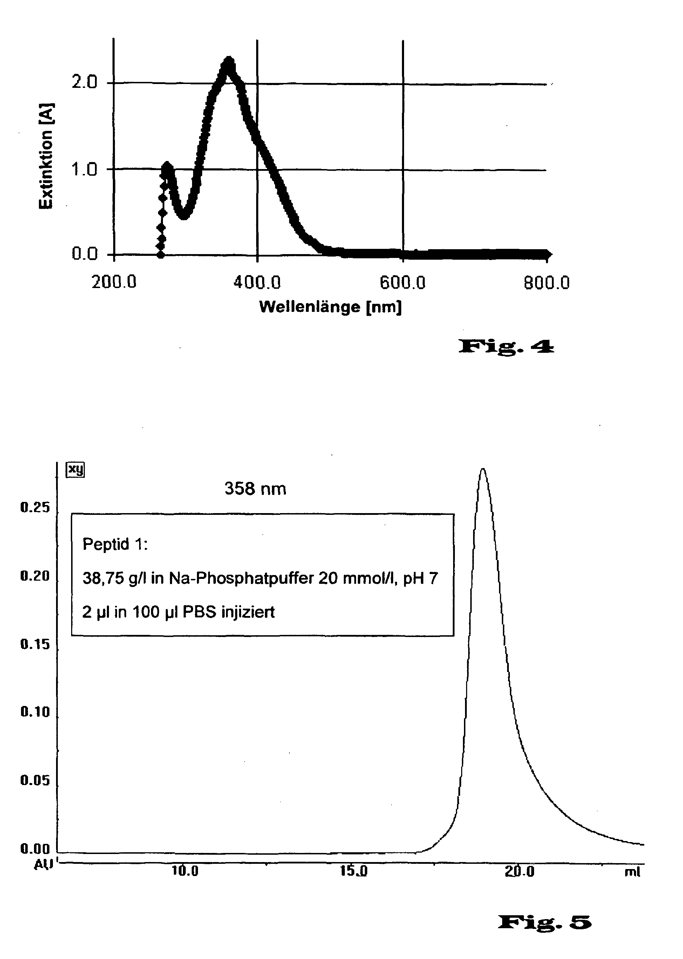

Mal-GKPLGL-Dap(Dnp)-AR-Doa-Cys(StBu)-NH₂ (Peptid 1; 0,39 mg/ml; 214 µmol/l) wurde durch Bestimmung des Absorptionsspektrums von 200-800 nm analysiert (Figur 4) Es wurden zwei Peaks bei 274 nm und 358 nm identifiziert. Die Extinktionskoeffizienten dieser beiden Absorptionsbanden belaufen sich auf: ε(358 nm)=10,5 mmol/L⁻¹cm⁻¹ und ε(274 nm)=4,3 mmol/L⁻¹cm⁻¹.

Das Peptid wurde weiterhin durch Gelfiltration analysiert. Dabei wurde die Absorption des Eluats bei 358 nm in einer Flusszelle gemessen (Figur 5).

### Beispiel 2: Synthese eines thiolfunktionalisierten niedermolekularen Peptids (Vergleichsbeispiel)

Es wurde ein niedermolekulares Vernetzerpeptid Ac-C-Doa-Doa-GKPLGL-Dap(Dnp)-AR-Doa-C-OH synthetisiert.

Mit PyClock™ als Aktivator wurde das Peptid an einem mit Fmoc-L-Cys(Trt)-OH beladenen "TCP" (Tritylchlorid-Polystyrol) -Harz (Pepchem) mittels der Festphasenpeptidsynthese-Technik auf einem "Prelude"-Peptidsynthesizer (Protein Technologies Inc., South Coach Drive, Tucson, AZ, USA) synthetisiert. Als Synthesebausteine wurden Fmoc-3,6-dioxaoktansäure und Fmoc-(2,4-dinitrophenyl)-diamino-propionsäure (Iris Biotech, Marktredwitz, Deutschland) sowie Fmoc-L-Arg(Pbf), Fmoc-L-Cys(Trt), Fmoc-Gly, Fmoc-L-Lys(Boc), Fmoc-L-Pro, Fmoc-L-Ala und Fmoc-L-Leu (Merck KGaA, Darmstadt, D) eingesetzt. Als Lösemittel wurde N,N-Dimethylformamid (Biosolve, Valkenswaard, NL) benutzt. Die N-terminale Acetyl-Gruppe wurde mittels einer Mischung aus Acetanhydrid und N-Methylmorpholin in NMP eingeführt.

Das Peptidyl-Harz wurde 5-mal mit Dichlormethan gewaschen und im Stickstoffstrom getrocknet. Anschließend wurde die Abspaltung des Peptids vom Harz mittels folgender Mischung durchgeführt: Phenol, Ethandithiol, Thioanisol, Wasser, Triisopropylsilan, Trifluoressigsäure = 3,35 / 2,1 / 3,35 / 4,2 / 2,9 / 84,1 (w/v/v/v/v/v).

Nach 2 Std. Schütteln des Harzes mit der genannten Mischung bei RT wurde das Peptid mit Diethylether ausgefällt, 2 Std. bei -20 °C aufbewahrt und das Präzipitat anschließend dreimal mit Diethylether gewaschen. Nach dem letzten Waschschritt wurde das Peptid mit Tert-Butanol / Wasser = 4 / 1 (v/v) aufgelöst und im Hochvakuum gefriergetrocknet.

Die Charakterisierung des Produkts mit HPLC und Massenspektrometrie ergab eine Reinheit von 83,4 % (Detektion bei 214 nm); die gefundene Masse ist [M+H]+ = 1746,36 im Vergleich zur monoisotopischen Sollmasse von 1745,81 .

### Beispiel 3: Konjugation des maleimid-/thiolfunktionalisierten synthetischen Peptids mit Dithiol-PEG

Es wird ein Peptid, Molekülmasse 0,5 kDa bis 2 kDa, hergestellt, das an einem Ende eine Maleimidgruppe trägt und am anderen Ende eine geschützte Thiolgruppe. Ein solches Peptid ist in Beispiel 1 beschrieben. Das Peptid wird mit Polyethylenglykol (PEG) mit einer mittleren Molekülmasse von 3 kDa bis 20 kDa, das an beiden Enden eine Thiolgruppe trägt, mit annähernd äquimolaren Konzentrationen versetzt. Dabei werden Reaktionsbedingungen gewählt, in denen eine oder beide Thiolgruppen des Polymers mit der Maleimidgruppe des Peptids konjugieren. Anschließend wird die Schutzgruppe am Thiol des Peptids entfernt und das Konjugat gereinigt.

Die Reaktion ist in den Figuren 1A und 1C und der dazugehörigen Figurbeschreibung schematisch dargestellt.

Als Resultat der Reaktion erhält man drei Polymerspezies:
*Thiol-*PEG*-Thiol* (kein Konjugat)
*Thiol-*PEG*-*Peptid*-Thiol* (1:1-Konjugat)
*Thiol*-Peptid-PEG-Peptid-*Thiol* (2:1-Konjugat)

Alle drei Polymere enthalten zwei endständige Bindungsfunktionen (hier: Thiolgruppen), deren Abstand voneinander durch die Summe der Molekülmassee des PEG- und Peptidanteils definiert ist.

HS-PEG-SH wurde durch Bestimmung der Thiolgruppen (Ellman-Test) und durch Bestimmung des PEG-Gehalts mit Polymethacrylat analysiert.

Peptid 1 aus Beispiel 1 (53,2 mg; 30,4 µmol; 1 Äquivalent) wurde in 3,3 ml Ammoniumacetat (100 mmol/L; pH 7,8) gelöst, mit HS-PEG-SH (33,4 µmol SH-Gruppen; 1,1 Äquivalent) vermischt und auf ein Gesamtvolumen von 5,3 ml mit Ammoniumacetat (100 mmol/L; pH 7,8) eingestellt. Nach einer Inkubation von 1 Std. auf Eis wurde ein Reduktionsmittel (Tris(2-carboxyethyl)-Phosphin (TCEP, 112,5 µmol)) zu der Lösung gegeben. Nach weiteren 40 min bei RT wurde zur der Lösung Essigsäure gegeben, bis ein pH von 2 erreicht war. Diese Lösung wurde in einen Dialyseschlauch (MWCO 2000) gefüllt und viermal gegen Phosphatpuffer (pH 3) mit absteigender Konzentration dialysiert (50 mmol/L, 2 mmol/L, 2 mmol/L und 0,2 mmol/L Phosphat). Das Dialysat (16,5 ml) wurde durch Sublimation auf ein Volumen von 1,84 ml reduziert, sterilfiltriert und in Aliquots bei -80 °C gelagert.

Die Konzentration und Ausbeute der Komponenten des PEG-Peptid-Konjugats (K1) wurde durch Bestimmung der Absorption von Dap(Dnp) (siehe Beispiel 1) und durch Bestimmung des PEG-Gehalts bestimmt:

| Komponente: | Peptid | PEG |
|---|---|---|
| Konzentration [mmol/L]: | 11,4 | 8,7 |
| Stoffmenge [µmol] (bei 1,84 ml): | 21 µmol | 16 µmol |
| Ausbeute: | 69% | 96% |

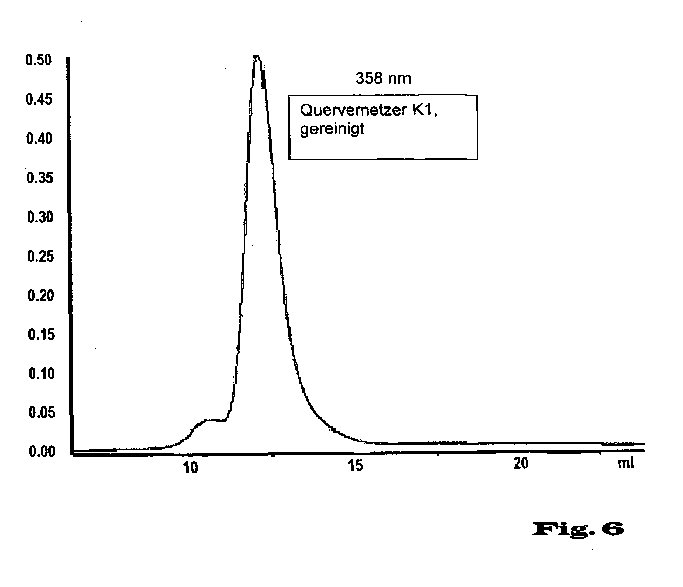

K1 wurde durch Gelfiltration und Erstellung eines Chromatogramms bei 358 nm analysiert (Figur 6). Dabei ergab sich eine Verschiebung des Dinitrophenylsignals (Dap(Dnp) von 19 ml (siehe Figur 5) auf 12,5 ml). Der Chromophor ist nach der Konjugationsreaktion also mit einer erheblich höheren Molekülmasse assoziiert.

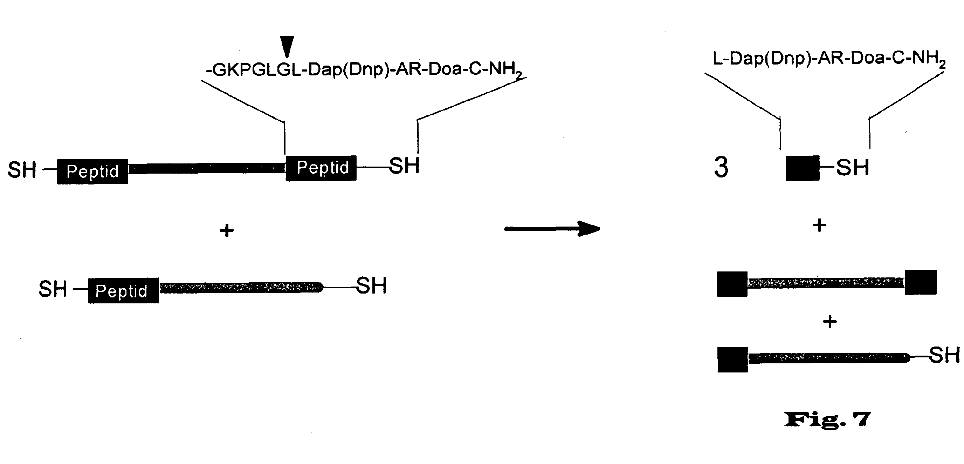

Die Spaltbarkeit des Peptids wurde mit MMP2 (Gelatinase) getestet. Dazu wurden 5 µl des Konjugats mit 2 µl MMP2 (Sigma Art.-Nr M9445) in 55 mmol/L Tris, 1,1 mmol/L CaCl₂, 0,055% Triton X-100, 2,9 mmol/L TCEP bei pH 7,5 in einem Gesamtvolumen von 110 µl für 1 Std. bei 37 °C inkubiert, um das Konjugat enzymatisch zu spalten. Aufgrund der Positionen der Schnittstelle für MMP2 auf Peptid 1 (zwischen Glycin und Leucin) und des Chromophors sollte durch den proteolytischen Verdau ein kleines Peptidfragment entstehen, welches den Chromophor enthält (Figur 7).

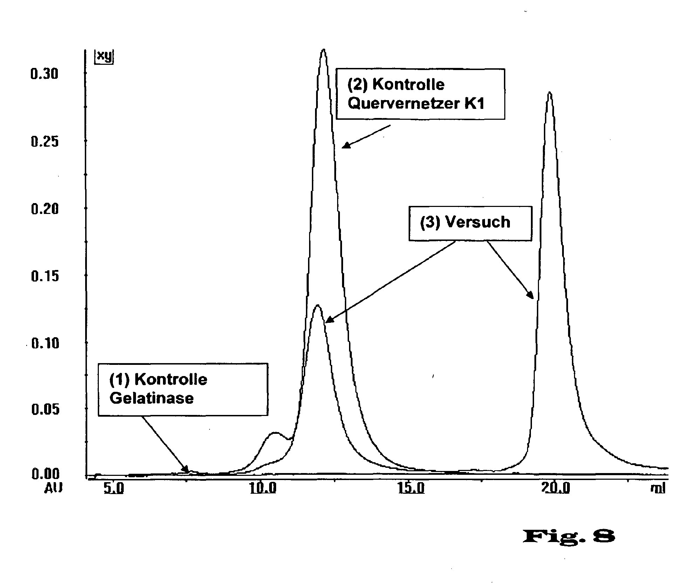

Zur Analyse der Abbaufragmente wurde der Ansatz zusammen mit Kontrollen (Enzym, unverdautes Konjugat) einer Gelfiltration unterzogen (Figur 8). Als mobile Phase wurde 100 mmol/L Ammoniumacetatpuffer (pH 4,8) mit 0,1 mmol/L TCEP eingesetzt, um die Oxidation der SH-Gruppen zu vermeiden (Figur 8). Die Gelfiltrationsanalyse zeigt, dass durch MMP2 von dem Konjugatmolekül ("Assay 3" in Figur 8) ein kleineres Fragment abgespalten wurde, welches bei etwa 20 ml eluiert.

Zur Analyse der Gelbildung wurde das Peptid-PEG-Konjugat (K1) mit zwei bekannten Vernetzern, die jeweils zwei distale Thiolgruppen enthielten und eine unterschiedliche Molekülmasse aufwiesen, verglichen. Die Vergleichsvernetzer waren Ac-Cys-Doa-Doa-KPLGL-Dpa-AR-Doa-Cys-OH (Peptid aus Beispiel 2) mit einer Molekülmasse von 1746 g/mol und HS-PEG-SH (10 kDa). Alle Vernetzer wurden dabei in unterschiedlichen Konzentrationen mit maleimidmodifiziertem Dextran vermischt, wobei Maleimidgruppen und Thiolgruppen in gleich hoher Menge verwendet wurden.

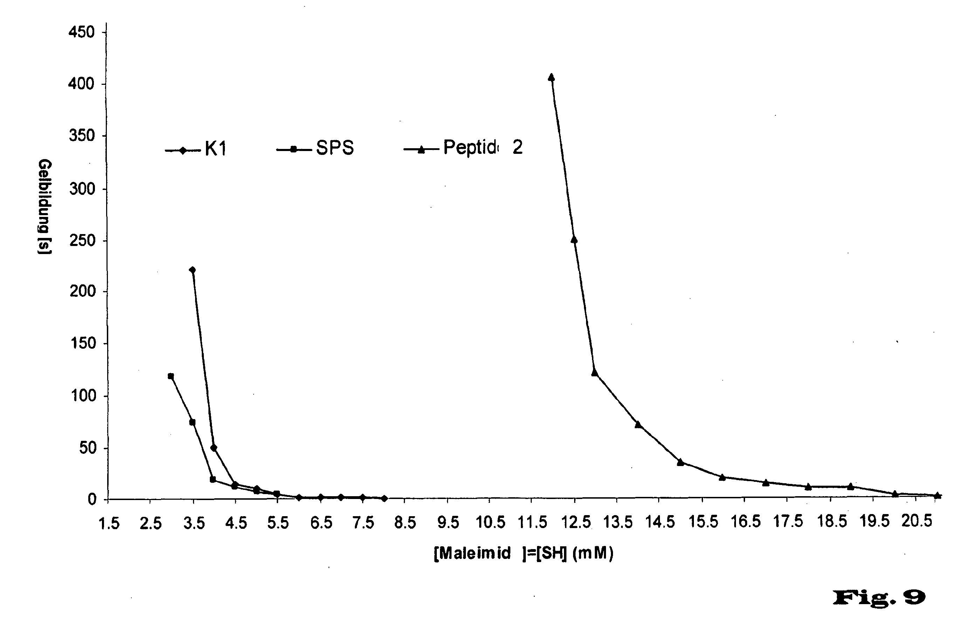

Figur 9 zeigt die Zeit bis zur Gelbildung, die durch Abschätzen der Viskosität der Ansätze ermittelt wurde.

Dabei zeigte sich, dass das Konjugat mit einer Molekülmasse von etwa 11500 g/mol, ähnlich wie HS-PEG-SH, ab einer Konzentration der reaktiven Gruppen von etwa 3-4 mmol/L Gele bildet. Im Gegensatz dazu bildet das niedermolekulare Peptid aus Beispiel 2, dass dieselbe biologisch relevante Sequenz wie K1 enthält, aber mit zwei distalen Thiolgruppen (Cystein) ausgestattet ist, erst ab etwa 13 mmol/L Gele. Damit wurde die Effizienz der Gelbildung durch das Peptid-PEG-Konjugat im Vergleich zum unkunjugierten Peptid um mehr als das 3 bis 4fache verbessert.

### Beispiel 4: In vitro Kultivierung von Fibroblasten

Hydrogele wurden mit einem Volumen von je 30 µl hergestellt und enthielten jeweils ca. 1,5x10⁴ 3T3 Zellen. Je nach Zusammensetzung der Gele wurden jeweils 5 mmol/L Maleimidgruppen des PVA, 1 mmol/L RGD-Peptid (Ac-C-Doa-Doa-GRGDSP-NH2) bzw. Thioglycerol, und eine 4 mmol/L entsprechende Menge an SH-Gruppen, die an das PEG-Peptid-Konjugats bzw. PEG (10 kDa) als Bindungsfunktionen gekoppelt sind, eingesetzt. Die Gele wurden wie in den Ausführungsbeispielen der DE 10 2007 034 580 A1 beschrieben hergestellt. Die ausgesäten Zellen mit DMEM (Dulbecco's Modified Eagle Medium, high glucose), 2 mmol/L Glutamin und 10 % fötalem Kälberserum bei 37 °C und 5 % CO₂ inkubiert.

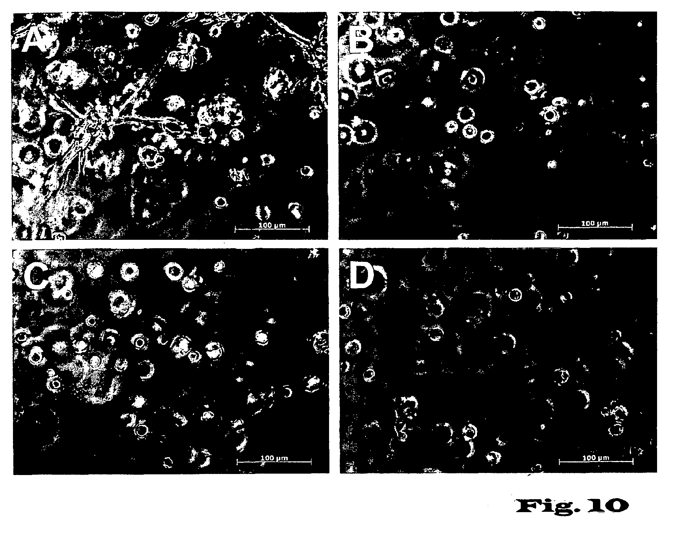

Die Figuren 10ABCD zeigen die Morphologie von Fibroblasten (Zelllinie 3T3) in PVA-Hydrogelen nach zwei Tagen in Kultur. 10A bis D sind mikroskopische Phasenkontrastbilder aus der Mitte des jeweiligen Gels aufgenommen und zeigen repräsentativ das Verhalten der Zellen im ganzen Gel. Hydrogele wurden mit MMP-spaltbarem Peptid-PEG Konjugat (10A, C) oder mit PEG (10B, D) als Vernetzungsreagenz hergestellt. In 10A und 10B wurde zudem in das Hydrogel 1 mmol/L des Adhäsionspeptids RGD, in 10C und 10D 1 mmol/L Thioglycerol kovalent an PVA angekoppelt. Die Fibroblasten nehmen nur dann eine spindelförmige Morphologie an, wenn die Hydrogele mit MMP-spaltbarem Vernetzer (Peptid-PEG Konjugat) und dem Adhäsionspeptid RGD hergestellt wurden (10A). Die Zellen bleiben rund, wenn der Vernetzer (PEG) nicht spaltbar ist, auch wenn ein geeignetes Zelladhäsionsmolekül (RGD) im Gel vorhanden ist (10B). In 10C ist gezeigt, dass trotz spaltbarem Vernetzer ohne Adhäsionsmoleküle keine spindelförmige Morphologie ausgebildet werden kann. Auch in Gelen ohne Adhäsionsmoleküle und spaltbarem Vernetzer bleiben die Zellen rund (10D).

## Patentansprüche

1. Verwendung von bindungsfunktionalisiertem Polymervernetzer mit jeweils im Bereich der Molekülenden des Vernetzers lokalisierten Bindungsfunktionen, der mindestens eine Peptidkomponente und mindestens eine damit linear verbundene hochmolekulare lineare Polymerkomponente und eine Molekülmasse von 3 kDa oder mehr aufweist, zur Vernetzung komplementär bindungsfunktionalisierter Polymere zu einem zwei- oder mehrkomponentigen Hydrogel.

2. Verwendung nach Anspruch 1, wobei die Polymerkomponente des Vernetzers eine Molekülmasse von 3 bis 50 kDa aufweist.

3. Verwendung nach Anspruch 1 oder 2, wobei die Peptidkomponente des Vernetzers eine Molekülmasse von 0,3 bis 10 kDa aufweist.

4. Verwendung nach einem der vorstehenden Ansprüche, wobei Polymerkomponente und Peptidkomponente in dem Vernetzer kovalent konjugiert sind.

5. Verwendung nach einem der vorstehenden Ansprüche, wobei der Vernetzer ein Gemisch ist aus Verbindungen, worin Polymerkomponente und Peptidkomponente in Molverhältnissen von 2:1, 1:1 und/oder 1:2 verbunden sind.

6. Verwendung nach einem der vorstehenden Ansprüche, wobei die Bindungsfunktionen zur Vernetzung ausgewählt sind aus der Gruppe kovalenter Bindungsfunktionen, bestehend aus: Carboxyl, Hydroxyl, Amin, Thiol, Maleimid, Vinylsulfon, Methacrylat, Acrylat, Acrylamid, Bromacetyl, Aldehyd, Amino, Aminoxy, Phosphin, Azid, Alkyn und Cyclooctin.

7. Verwendung nach einem der vorstehenden Ansprüche, wobei die hochmolekulare lineare Polymerkomponente des Vernetzers ausgewählt ist aus: PEG, PPG, Block-Copolymeren daraus sowie jeweils Mischungen von zwei oder mehreren davon.

8. Verwendung nach einem der vorstehenden Ansprüche, wobei der Vernetzer zwischen den jeweils im Bereich der Molekülenden lokalisierten Bindungsfunktionen mindestens eine spaltbare intramolekulare Verbindung aufweist, die ausgewählt ist aus: wasserspaltbaren Verbindungen, enzymspaltbaren Verbindungen, pH-abhängigen Verbindungen, temperaturabhängigen Verbindungen und elektromagnetisch (Mikrowelle, Licht, UV, IR) spaltbaren Verbindungen.

9. Verwendung nach Anspruch 8, wobei die spaltbare Verbindung eine enzymspaltbare Aminosäureabfolge in mindestens einer Peptidkomponente des Vernetzers ist.

10. Verfahren zur Herstellung eines Hydrogels, enthaltend die Schritte:
- Bereitstellen eines in einem der Ansprüche 1 bis 9 charakterisierten bindungsfunktionalisierten Polymervernetzers,
- Bereitstellen einer Mehrzahl komplementär bindungsfunktionalisierter Polymere und
- Inkontaktbringen des Polymervernetzers mit den Polymeren unter Bedingungen, die eine Konjugation der Bindungsfunktionen des Polymervernetzers mit den komplementären Bindungsfunktionen der Polymere ermöglichen, sodass ein zwei- oder mehrkomponentiges Hydrogel aus einer Mehrzahl von mit Polymervernetzer vernetzter Polymere gebildet wird.

11. Verfahren nach Anspruch 10, wobei der Anteil an bereitgestelltem Polymervernetzer weniger als 10 mmol/L der Bindungsfunktionen, bezogen auf das hergestellte Hydrogel, beträgt.

12. Verfahren nach Anspruch 10 oder 11, wobei zur Herstellung des Polymervernetzers mit jeweils im Bereich von dessen beiden Molekülenden lokalisierter Bindungsfunktionen eines ersten Typs (A) zur Vernetzung von Polymeren, die zu der Bindungsfunktion des ersten Typs (A) komplementäre Bindungsfunktionen eines zweiten Typs (B) aufweisen, zumindest die Schritte
- Bereitstellen einer ersten Komponente, die eine im Bereich von deren einen Molekülende lokalisierte Bindungsfunktion des ersten Typs (A) und eine im Bereich von deren anderen Molekülende lokalisierte Bindungsfunktion eines dritten Typs (C) aufweist, wobei die erste Komponente entweder
(a) ein in den Ansprüchen 1 bis 9 charakterisiertes Polymermolekül oder
(b) ein in den Ansprüchen 1 bis 9 charakterisiertes Peptidmolekül ist;
- Bereitstellen einer zweiten Komponente mit jeweils im Bereich von deren beiden Molekülenden lokalisierten Bindungsfunktionen eines vierten Typs (D), die zu der Bindungsfunktion des dritten Typs (C) komplementär sind, wobei die zweite Komponente das jeweils andere Molekül gemäß (a) oder (b) ist; und
- Inkontaktbringen der ersten Komponente mit der zweiten Komponente unter Bedingungen, die die lineare Konjugation der Bindungsfunktion des dritten Typs (C) der ersten Komponente mit der Bindungsfunktion des vierten Typs (D) der zweiten Komponente ermöglichen, so dass als Polymervernetzer ein lineares Konjugat mit jeweils im Bereich der Molekülenden des Konjugats lokalisierter Bindungsfunktion vom Typ (A) gebildet wird;
durchgeführt werden.

13. Verfahren nach Anspruch 10 oder 11, wobei zur Herstellung des Polymervernetzers mit jeweils im Bereich von dessen beiden Molekülenden lokalisierter Bindungsfunktionen eines ersten Typs (A) zur Vernetzung von Polymeren, die zu der Bindungsfunktion des ersten Typs (A) komplementäre Bindungsfunktionen eines zweiten Typs (B) aufweisen, zumindest die Schritte
- Bereitstellen einer ersten Komponente, die eine im Bereich von deren einen Molekülende lokalisierte Bindungsfunktion des ersten Typs (A) und eine im Bereich von deren anderen Molekülende lokalisierte Bindungsfunktion eines dritten Typs (C) aufweist, wobei die erste Komponente entweder
(a) ein in den Ansprüchen 1 bis 9 charakterisiertes Polymermolekül oder
(b) ein in den Ansprüchen 1 bis 9 charakterisiertes Peptidmolekül ist;
- Bereitstellen einer zweiten Komponente, die eine im Bereich von deren einen Molekülende lokalisierte Bindungsfunktion des ersten Typs (A) und eine im Bereich von deren anderen Molekülende lokalisierte Bindungsfunktion eines vierten Typs (D), die zu der Bindungsfunktion des dritten Typs (C) komplementär ist, aufweist, wobei die zweite Komponente das jeweils andere Molekül gemäß (a) oder (b) ist; und
- Inkontaktbringen der ersten Komponente mit der zweiten Komponente unter Bedingungen, die die lineare Konjugation der Bindungsfunktion des dritten Typs (C) der ersten Komponente mit der Bindungsfunktion des vierten Typs (D) der zweiten Komponente ermöglichen, so dass als Polymervernetzer ein lineares Konjugat mit jeweils im Bereich der Molekülenden des Konjugats lokalisierter Bindungsfunktion vom Typ (A) gebildet wird;
durchgeführt werden.

14. Verfahren nach Anspruch 10 oder 11, wobei zur Herstellung eines Polymervernetzers mit jeweils im Bereich von dessen beiden Molekülenden lokalisierter Bindungsfunktionen eines ersten Typs (A) zur Vernetzung von Polymeren, die zu der Bindungsfunktion des ersten Typs (A) komplementäre Bindungsfunktionen eines zweiten Typs (B) aufweisen, zumindest die Schritte
- Bereitstellen einer ersten Komponente mit jeweils im Bereich von deren beiden Molekülenden lokalisierten Bindungsfunktionen des ersten Typs (A), wobei die erste Komponente entweder
(a) ein in den Ansprüchen 1 bis 9 charakterisiertes Polymermolekül oder
(b) ein in den Ansprüchen 1 bis 9 charakterisiertes Peptidmolekül ist;
- Bereitstellen einer zweiten Komponente mit einer im Bereich von deren einen Molekülende lokalisierten Bindungsfunktion des ersten Typs (A) und mit einer im Bereich von deren anderen Molekülende lokalisierten Bindungsfunktion des zweiten Typs (B), wobei die zweite Komponente das jeweils andere Molekül gemäß (a) oder (b) ist; und
- Inkontaktbringen der ersten Komponente mit der zweiten Komponente unter Bedingungen, die die lineare Konjugation einer Bindungsfunktion des ersten Typs (A) der ersten Komponente mit der Bindungsfunktion des zweiten Typs (B) der zweiten Komponente ermöglichen, so dass als Polymervernetzer ein lineares Konjugat oder Konjugat-Gemisch mit jeweils im Bereich der beiden Molekülenden des Konjugats lokalisierter Bindungsfunktion des ersten Typs (A) gebildet wird;
durchgeführt werden.

15. Verfahren nach Anspruch 14, wobei in dem Schritt des Bereitstellens des Peptids die Bindungsfunktion des ersten Typs (A) der zweiten Komponente eine Schutzgruppe zur Unterdrückung von Selbstkonjugation mit der Bindungsfunktion (B) aufweist, und wobei die Schutzfunktion während des Inkontaktbringens der ersten Komponente mit der zweiten Komponente Peptid entfernt wird.

16. Verfahren nach einem der Anspruche 12bis 15, wobei die erste Komponente das Polymermolekül und die zweite Komponente das Peptidmolekül ist.

17. Verfahren nach einem der Anspruche 12bis 15, wobei die erste Komponente das Peptidmolekül und die zweite Komponente das Polymermolekül ist.

18. Hydrogel, herstellbar nach dem Verfahren nach einem der Ansprüche 10 bis 17.

19. Hydrogel nach Anspruch 18, enthaltend, darin gebunden, den Polymervernetzer in einem Anteil von weniger als 10 mmol/L der Bindungsfunktionen.

20. Hydrogel nach Anspruch 18oder 19, das ein Zellkulturgel ist.

21. Verwendung des in einem der Ansprüche 1 bis 9 charakterisierten Polymervernetzers zur Steigerung des Wasseranteils in Hydrogelen.

22. Kit zur Herstellung eines zwei- oder mehrkomponentigen Hydrogels, enthaltend:
- bindungsfunktionalisierter Polymervernetzer charakterisiert in einem der Ansprüche 1 bis 9 und
- komplementär bindungsfunktionalsiertes Polymer.

## Claims

1. Use of bond functionalized polymer crosslinking agent with bond functions which are each localized in the area of the molecular termini of the crosslinking agent, having at least one peptide component and at least one high-molecular linear polymer component bonded to the at least one peptide component to form a linear molecule and having a molecular weight of 3 kDa or more, for crosslinking complementary bond functionalized polymers to form a two-component or multicomponent hydrogel.

2. Use according to Claim 1, wherein the polymer component of the crosslinking agent has a molecular weight of 3 to 50 kDa.

3. Use according to Claim 1 or 2, wherein the peptide component of the crosslinking agent has a molecular weight of 0.3 to 10 kDa.

4. Use according to any one of the preceding claims, wherein the polymer component and peptide component are covalently conjugated in the crosslinking agent.

5. Use according to any one of the preceding claims, wherein the crosslinking agent is a mixture of compounds wherein the polymer component and the peptide component are bonded in molar ratios of 2:1, 1:1 and/or 1:2.

6. Use according to any one of the preceding claims, wherein the bond functions for crosslinking are selected from the group of covalent bond functions consisting of carboxyl, hydroxyl, amine, thiol, maleimide, vinylsulfone, methacrylate, acrylate, acrylamide, bromoacetyl, aldehyde, amino, aminoxy, phosphine, azide, alkyne and cyclooctyne.

7. Use according to any one of the preceding claims, wherein the high-molecular linear polymer component of the crosslinking agent is selected from PEG, PPG, block copolymers thereof and mixtures of two or more thereof.

8. Use according to any one of the preceding claims, wherein the crosslinking agent has at least one cleavable intramolecular bond between the bond functions localized in the area of the molecular termini, this cleavable intramolecular bond being selected from: water-cleavable compounds, enzyme-cleavable compounds, pH-dependent compounds, temperature-dependent compounds and electromagnetically (microwave, light, UV, IR) cleavable compounds.

9. Use according to Claim 8, wherein the cleavable bond is an enzyme-cleavable amino acid sequence in at least one peptide component of the crosslinking agent.

10. Method for producing a hydrogel, comprising the steps:
- providing a bond functionalized polymer crosslinking agent, **characterized in** one of Claims 1 to 9,
- providing a plurality of complementary bond functionalized polymers and
- bringing the polymer crosslinking agent in contact with the polymers under conditions which enable conjugation of the bond functions of the polymer crosslinking agent with the complementary bond functions of the polymer, so that two component or multicomponent hydrogel is formed from a plurality of polymers crosslinked with polymer crosslinking agents.

11. Method according to claim 10, wherein the amount of polymer crosslinking agent provided amounts to less than 10 mmol/liter of the bond functions, based on the hydrogel produced.

12. Method according to claim 10 or 11, wherein to produce the polymer crosslinking agent with bond functions of a first type (A) localized in the area of its two molecular termini for crosslinking of polymers which have bond functions of a second type (B) that are complementary to the bond function of the first type (A), at least the following steps are performed:
- providing a first component which has a bond function of the first type (A) localized in the area of its one molecular terminus and a bond function of a third type (C) localized in the area of its other molecular terminus, wherein the first component is either
(a) a polymer molecule **characterized in** Claims 1 to 9 or
(b) a peptide molecule **characterized in** Claims 1 to 9;
- providing a second component having bond functions of a fourth type (D) localized in the area of its two molecular termini, these bond functions being complementary to the bond function of the third type (C) wherein the second component is the other respective molecule according to (a) or (b); and
- bringing the first component in contact with the second component under conditions which enable linear conjugation of the bond function of the third type (C) of the first component with the bond function of the fourth type (D) of the second component so that a linear conjugate with a bond function of type (A) localized in the area of the molecular termini is formed as the polymer crosslinking agent.

13. Method according to claim 10 or 11, wherein to produce the polymer crosslinking agent with bond functions of a first type (A) localized in the area of its two molecular termini for crosslinking of polymer having bond functions of a second type (B) complementary to the bond functions of the first type (A), at least the following steps are performed:
- providing a first component which has a bond function of the first type (A) localized in the area of its one molecular terminus and having a bond function of a third type (C) localized in the area of its other molecular terminus, wherein the first component is either
(a) a polymer molecule **characterized in** Claims 1 to 9 or
(b) a peptide molecule **characterized in** Claims 1 to 9;
- providing a second component having a bond function of a first type (A) localized in the area of its one molecular terminus and having a bond function of a fourth type (D) localized in the area of the other molecular terminus, this fourth type being complementary to the bond function of the third type (C), wherein the second component is the other respective molecule according to (a) or (b); and
- bringing the first component in contact with the second component under conditions which enable linear conjugation of the bond function of the third type (C) of the first component having the bond function of the fourth type (D) of the second component, so that a linear conjugate with a bond function of type (A) localized in the area of the molecular termini of the conjugate is formed as the polymer crosslinking agent.

14. Method according to claim 10 or 11, wherein at least the following steps are performed to synthesize a polymer crosslinking agent having bond functions of a first type (A) localized in the area of its two molecular termini for crosslinking of polymers having bond functions of a second type (B) complementary to the bond functions of the first type (A):
- providing a first component having bond functions of the first type (A) localized in the area of its two molecular termini, wherein the first component is either
(a) a polymer molecule **characterized in** Claims 1 to 9 or
(b) a peptide molecule **characterized in** Claims 1 to 9;
- providing a second component having a bond function of a first type (A) localized in the area of its one molecular terminus and a bond function of the second type (B) localized in the area of the other molecular terminus, wherein the second component is the other respective molecule according to (a) or (b); and
- bringing the first component in contact with the second component under conditions which enable linear conjugation of a bond function of the first type (A) of the first component having the bond function of the second type (B) of the second component, so that a linear conjugate or conjugate mixture having a bond function of the first type (A) localized in the area of each of the two molecular termini of the conjugate is formed as the polymer crosslinking agent.

15. Method according to claim 14, wherein, in the step of providing the peptide, the bond function of the first type (A) of the second component has a protective group for suppressing self-conjugation with the bond function (B), and wherein the protective function is removed when the first component is brought in contact with the second component, the peptide.

16. Method according to any one of claims 12 to 15, wherein the first component is the polymer molecule and the second component is the peptide molecule.

17. Method according to any one of claims 12 to 15, wherein the first component is the peptide molecule and the second component is the polymer molecule.

18. Hydrogel synthesizable by the method according to any one of claims 10 to 17.

19. Hydrogel according to claim 18 comprising the polymer crosslinking agent in an amount of less than 10 mmol/liter of the bond functions bound into the hydrogel.

20. Hydrogel according to claim 18 or 19 which is cell culture gel.

21. Use of the polymer crosslinking agent **characterized in** any one of claims 1 to 9 to increase the water content in hydrogels.

22. Kit to produce a two-component or multicomponent hydrogel comprising
- bond functionalized polymer crosslinking agent **characterized in** any one of Claims 1 to 9 and
- complementary bond functionalized polymer.

## Revendications

1. Utilisation d'un agent réticulant des polymères à fonctionnalité de liaison, respectivement ayant des fonctionnalités de liaison localisées au niveau des extrémités de molécule de l'agent réticulant, l'agent réticulant présentant au moins un composant peptidique et au moins un composant polymérique linéaire à masse moléculaire élevé et linéairement lié au composant peptidique, et un masse moléculaire de 3 kDa ou plus, pour la réticulation des polymères à fonctionnalité de liaison complémentaire afin d'obtenir un hydrogel à deux ou plusieurs composants.

2. Utilisation selon la revendication 1, le composant polymérique de l'agent réticulant présentant une masse moléculaire de 3 à 50 kDa.

3. Utilisation selon la revendication 1 ou 2, le composant peptidique de l'agent réticulant présentant une masse moléculaire de à 0,3 à 10 kDa.

4. Utilisation selon l'une quelconque des revendications précédentes, le composant polymérique et le composant peptidique étant conjugués de façon covalente dans l'agent réticulant.

5. Utilisation selon l'une quelconque des revendications précédentes, l'agent réticulant étant un mélange des composés, dans lesquels le composant polymérique et le composant peptidique sont liés dans des rapports molaires de 2:1, 1:1 et/ou 1:2.

6. Utilisation selon l'une quelconque des revendications précédentes, dans laquelle les fonctionnalités de liaison pour la réticulation sont choisies dans le groupe des fonctionnalités covalentes de liaison, consistant en: carboxyle, hydroxyle, amine, thiol, maléimide, vinylsulfone, méthacrylate, acrylate, acrylamide, bromoacétyle, aldéhyde, amino, aminoxy, phosphine, azide, alcyne et cyclooctine.

7. Utilisation selon l'une quelconque des revendications précédentes, le composant polymérique linéaire à masse moléculaire élevé de l'agent réticulant étant choisi parmi: PEG, PPG, copolymères séquencés, et mélanges respectifs de deux ou plusieurs de ceux-ci.

8. Utilisation selon l'une quelconque des revendications précédentes, dans laquelle l'agent réticulant présente, entre les fonctionnalités de liaison respectivement localisées au niveau des extrémités des molécules, au moins une liaison intramoléculaire clivable qui est choisie parmi: liaisons clivables par l'eau, liaisons clivables par une enzyme, liaisons dépendantes du pH, liaisons dépendantes de la température et liaisons clivables éléctromagnétiquement (microonde, lumière, UV, IR).

9. Utilisation selon la revendication 8, la liaison clivable étant une séquence d'acides aminés, clivable par une enzyme, dans au moins un composant peptidique de l'agent réticulant.

10. Procédé pour la fabrication d'un hydrogel, le procédé contenant les étapes suivantes :
- fournir un agent réticulant des polymères à fonctionnalité de liaison comme caractérisé selon l'une quelconque des revendications 1 à 9,
- fournir une pluralité des polymères à fonctionnalité de liaison complémentaire, et
- mettre en contact l'agent réticulant des polymères avec les polymères, dans des conditions qui permettent une conjugaison des fonctionnalités de liaison de l'agent réticulant des polymères avec les fonctionnalités de liaison complémentaires des polymères, afin de former un hydrogel à deux ou plusieurs composants à partir d'une pluralité des polymères réticulés avec l'agent réticulant des polymères.

11. Procédé selon la revendication 10, dans lequel la proportion de l'agent réticulant des polymères fournie est moins de 10 mmol/L des fonctionnalités de liaison par rapport à l'hydrogel fabriqué.

12. Procédé selon la revendication 10 ou 11, dans lequel, pour la fabrication de l'agent réticulant des polymères avec des fonctionnalités de liaison d'un premier type (A) localisées respectivement au niveau de ses deux extrémités de molécule, pour la réticulation des polymères qui présentent des fonctionnalités de liaison d'un second type (B) complémentaires à la fonctionnalité de liaison du premier type (A), au moins les étapes
- fournir un premier composant qui présente une fonctionnalité de liaison de premier type (A) localisée au niveau d'un de ses extrémités de molécule, et une fonctionnalité de liaison d'un troisième type (C) localisée au niveau de son autre extrémité de molécule, où le premier composant est
(a) une molécule de polymère **caractérisée** dans les revendications 1 à 9, ou
(b) une molécule de peptide **caractérisée** dans les revendications 1 à 9 ;
- fournir un second composant ayant, localisée respectivement au niveau de ses deux extrémités de molécule, des fonctionnalités de liaison d'un quatrième type (D) qui sont complémentaires à la fonctionnalité de liaison du troisième type (C), le second composant respectivement étant l'autre molécule selon (a) ou (b) ; et
- mettre en contact le premier composant avec le second composant, dans des conditions qui permettent la conjugaison linéaire de la fonctionnalité de liaison du troisième type (C) du premier composant avec la fonctionnalité de liaison du quatrième type (D) du second composant, afin de former, en tant que l'agent réticulant des polymères, un conjugué linéaire avec des fonctionnalités de liaison du type (A) respectivement localisées au niveau des extrémités de molécule du conjugué;
sont effectuées.

13. Procédé selon la revendication 10 ou 11, dans lequel, pour la fabrication de l'agent réticulant des polymères avec des fonctionnalités de liaison d'un premier type (A) localisées respectivement au niveau de ses deux extrémités de molécule, pour la réticulation des polymères qui présentent des fonctionnalités de liaison d'un second type (B) complémentaires à la fonctionnalité de liaison du premier type (A), au moins les étapes
- fournir un premier composant qui présente une fonctionnalité de liaison de premier type (A) localisée au niveau d'un de ses extrémités de molécule, et une fonctionnalité de liaison d'un troisième type (C) localisée au niveau de son autre extrémité de molécule, où le premier composant est
(a) une molécule de polymère **caractérisée** dans les revendications 1 à 9, ou
(b) une molécule de peptide **caractérisée** dans les revendications 1 à 9 ;
- fournir un second composant ayant une fonctionnalité de liaison du premier type (A) localisée au niveau d'un de ses extrémités de molécule, et une fonctionnalité de liaison d'un quatrième type (D), qui est complémentaire à la fonctionnalité de liaison du troisième type (C), localisée au niveau de son autre extrémité de molécule, le second composant respectivement étant l'autre molécule selon (a) ou (b) ; et
- mettre en contact le premier composant avec le second composant, dans des conditions qui permettent la conjugaison linéaire de la fonctionnalité de liaison du troisième type (C) du premier composant avec la fonctionnalité de liaison du quatrième type (D) du second composant, afin de former, en tant que l'agent réticulant des polymères, un conjugué linéaire avec des fonctionnalités de liaison du type (A) respectivement localisées au niveau des extrémités de molécule du conjugué;
sont effectuées.

14. Procédé selon la revendication 10 ou 11, dans lequel, pour la fabrication de l'agent réticulant des polymères avec des fonctionnalités de liaison d'un premier type (A) localisées respectivement au niveau de ses deux extrémités de molécule, pour la réticulation des polymères qui présentent des fonctionnalités de liaison d'un second type (B) complémentaires à la fonctionnalité de liaison du premier type (A), au moins les étapes
- fournir un premier composant avec des fonctionnalités de liaison du premier type (A) respectivement localisées au niveau de ses deux extrémités de molécule, où le premier composant est
(a) une molécule de polymère **caractérisée** dans les revendications 1 à 9, ou
(b) une molécule de peptide **caractérisée** dans les revendications 1 à 9 ;
- fournir un second composant avec une fonctionnalité de liaison du premier type (A) localisée au niveau d'un de ses extrémités de molécule, et une fonctionnalité de liaison d'un deuxième type (B) localisée au niveau de son autre extrémité de molécule, le second composant respectivement étant l'autre molécule selon (a) ou (b) ; et
- mettre en contact le premier composant avec le second composant, dans des conditions qui permettent la conjugaison linéaire d'une fonctionnalité de liaison du premier type (A) du premier composant avec la fonctionnalité de liaison du deuxième type (B) du second composant, afin de former, en tant que l'agent réticulant des polymères, un conjugué linéaire ou mélange de conjugués avec des fonctionnalités de liaison du premier type (A) respectivement localisées au niveau des deux extrémités de molécule du conjugué ;
sont effectuées.

15. Procédé selon la revendication 14, dans lequel dans l'étape de fournir le peptide, la fonctionnalité de liaison du premier type (A) du second composant présente un groupe protecteur pour prévenir l'auto-conjugaison avec la fonctionnalité de liaison (B), et dans lequel la fonctionnalité protecteur est éliminée pendant l'étape de mettre en contact le premier composant avec le second composant peptide.

16. Procédé selon l'une quelconque des revendications 12 à 15, dans lequel le premier composant est la molécule de polymère et le second composant est la molécule de peptide.

17. Procédé selon l'une quelconque des revendications 12 à 15, dans lequel le premier composant est la molécule de peptide et le second composant est la molécule de polymère.

18. Hydrogel qui peut être fabriqué par le procédé selon l'une quelconque des revendications 10 à 17.

19. Hydrogel selon la revendication 18, contenant, en forme liée, l'agent réticulant des polymères dans une proportion de moins de 10 mmol/L des fonctionnalités de liaison.

20. Hydrogel selon la revendication 18 ou 19 qui est un gel pour la culture cellulaire.

21. Utilisation de l'agent réticulant des polymères caractérisé dans l'une quelconque des revendications 1 à 9 pour l'augmentation de la teneur en eau dans les hydrogels.

22. Kit pour la fabrication d'un hydrogel à deux ou plusieurs composants, le kit contenant :
- un agent réticulant des polymères à fonctionnalité de liaison comme caractérisé selon l'une quelconque des revendications 1 à 9, et
- un polymère à fonctionnalité de liaison complémentaire.
